(19) Europäisches Patentamt
European Patent Office
Office européen des brevets

(11) EP 1 405 627 B1

(12) FASCICULE DE BREVET EUROPEEN

(45) Date de publication et mention de la délivrance du brevet:
**30.04.2008 Bulletin 2008/18**

(51) Int Cl.:
***A61K 8/86*** *(2006.01)* ***A61K 8/97*** *(2006.01)*
***A61Q 5/10*** *(2006.01)*

(21) Numéro de dépôt: **03292353.4**

(22) Date de dépôt: **25.09.2003**

(54) **Composition antipénétrante de prétraitement capillaire à base de polymère(s) d'oxyde d'éthylène et procédé utilisant une telle composition pour limiter la pénétration dans la peau de colorants**

Antipenetrante Haarvorbehandlungsmittel basiert auf Polyäthylenoxidpolymer(e) und Verfahren zur Beschränkung der Hautpenetration von Farbstoffen durch Verwendung dieses Mittels

Antipenetrating hair pretreatment composition based on polyethelenoxide polymer(s) and process using said composition for limiting skin penetration by colourants

(84) Etats contractants désignés:
**AT BE BG CH CY CZ DE DK EE ES FI FR GB GR HU IE IT LI LU MC NL PT RO SE SI SK TR**

(30) Priorité: **27.09.2002 FR 0212032**

(43) Date de publication de la demande:
**07.04.2004 Bulletin 2004/15**

(73) Titulaire: **L'ORÉAL**
**75008 Paris (FR)**

(72) Inventeur: **Dreher, Frank**
**San Francisco CA 94147 (US)**

(74) Mandataire: **Catherine, Alain et al**
**Cabinet Harlé & Phélip**
**7, rue de Madrid**
**75008 Paris (FR)**

(56) Documents cités:
**EP-A- 0 801 942**
**DE-A- 10 200 185**
**US-A- 3 933 422**
**US-A- 5 500 218**
**US-A- 5 534 245**
**US-B1- 6 235 273**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne d'une manière générale une composition anti-pénétrante de prétraitement capillaire comprenant une quantité efficace d'au moins un copolymère oxyéthyléné.

L'invention concerne également un procédé pour limiter la pénétration dans la peau et/ou les fibres kératiniques d'au moins un colorant, en particulier un colorant d'oxydation, contenu dans une composition de coloration capillaire, consistant à appliquer en prétraitement sur les cheveux une telle composition antipénétrante, avant le processus de coloration des cheveux.

Certains colorants, en particulier certains colorants d'oxydation, sont susceptibles de provoquer des réactions d'inconfort, d'irritation, de sensibilisation ou de tâchage du cuir chevelu, même si toutes les précautions sont prises pendant une coloration pour éviter le contact avec la peau, que leur possibilité d'être en contact avec la peau est très courte, et qu'ils sont rincés juste après coloration. De telles réactions d'intolérance sont déclenchées quand le colorant atteint ou dépasse la barrière cutanée.

Pour des colorants capillaires susceptibles de provoquer de telles réactions, une limitation du passage transcutané aurait pour conséquence une meilleure tolérance du colorant et permettrait d'éviter un tâchage de la peau.

On connaît de nombreuses familles de composés qui ont la propriété d'augmenter la pénétration dans la peau ou les fibres kératiniques d'un agent actif contenu dans une composition cosmétique et/ou pharmaceutique.

Par contre, on connaît très peu de familles de composés possédant l'activité inverse, c'est-à-dire réduisant effectivement la pénétration d'un ingrédient d'une composition cosmétique et/ou pharmaceutique dans la peau et/ou les fibres kératiniques.

Il serait souhaitable de disposer de composés ou formulations ayant des propriétés de réduction de la pénétration dans la peau et/ou les fibres kératiniques d'ingrédients de compositions cosmétiques et/ou pharmaceutiques.

En effet, pour certaines formulations cosmétiques pour application sur la peau et/ou les fibres kératiniques, il serait très souhaitable de réduire, retarder, voire supprimer la pénétration de l'ingrédient dans la peau et/ou les fibres kératiniques.

Il serait donc souhaitable de disposer d'un agent qui, utilisé en complément d'une composition cosmétique et/ou pharmaceutique de base contenant au moins un agent cosmétiquement et/ou thérapeutiquement actif, limite, voire supprime la pénétration dans la peau et/ou les fibres kératiniques de l'agent cosmétiquement et/ou thérapeutiquement actif.

En particulier, dans le cas de la coloration capillaire, il serait souhaitable de disposer d'un agent qui, utilisé en complément d'une composition cosmétique de coloration des fibres kératiniques, par exemple les cheveux, limiterait, voire supprimerait l'absorption cutanée et le passage transcutané du colorant sans nuire à la coloration des fibres kératiniques.

La demanderesse a maintenant découvert qu'il était possible de limiter l'absorption cutanée et le passage transcutané de colorants, notamment de colorants d'oxydation, contenus dans une composition de coloration capillaire, en particulier des paraphénylène diamines, en appliquant en prétraitement sur le cuir chevelu ainsi que sur les cheveux avant l'application de la composition de coloration, une composition antipénétrante à base de polymère(s) comprenant des fonctions oxyéthylénées.

En particulier, la demanderesse a découvert que l'utilisation d'au moins un polymère oxyéthyléné choisi parmi les polyéthylène glycol comprenant un nombre moyen de groupements oxydes d'éthylène supérieur à 45 et inférieur à 795, c'est-à-dire, de masse moléculaire supérieure à 2000 $g.mol^{-1}$ et inférieure à 35000 $g.mol^{-1}$, permettait de limiter le passage transcutané de colorants, notamment de colorants d'oxydation, contenus dans une composition de coloration capillaire.

L'homme de l'art connaît l'utilisation des polymères oxyéthylénés comme agents antipénétrants dans des compositions cosmétiques contenant au moins un actif, pour limiter, voire supprimer la pénétration de l'actif dans la peau.

De nombreuses publications scientifiques, ainsi que des brevets et demandes de brevet décrivent les effets anti-pénétrants de tels polymères. Ainsi, à titre de document illustratif, on peut citer les publications J. Pharm. Sci. 67 (1978), 517-520 ; J. Pharm. Sci. 68(1979), 596-598 ; et J. Controlled Release 23 (1993), 247-260.

On peut également citer les brevets américains US 5,650,171 et US 5,700,483 qui décrivent des compositions topiques contenant une quantité effective d'acide rétinoïque en tant qu'actif pour le traitement de la peau et un polyuréthane de masse moléculaire élevée (allant jusqu'à 60 000 pour US 5,650,171 et jusqu'à 200 000 pour US 5,700,483) pour diminuer le passage transcutané de l'acide rétinoïque ; le brevet américain US 5,989,527 qui décrit des compositions topiques destinées à être appliquées sur le stratum corneum, contenant un actif (tel que par exemple un agent chimique exfoliant, un agent autobronzant, un agent éclaircissant pour la peau et/ou un agent insectifuge) et un polyester pour retenir l'actif dans le stratum corneum empêchant ainsi son passage transcutané ; le brevet américain US 5,833,961 qui décrit des compositions solaires résistantes à l'eau et à la transpiration, contenant au moins deux filtres solaires organiques en tant qu'actifs et des polyesters fonctionnalisés pour fixer et maintenir les actifs dans le stratum corneum ; le brevet américain US 4,938,951 qui décrit des compositions liquides topiques, contenant un actif topique (tel que par

exemple un agent absorbant la lumière ultra-violette, un insecticide, un pesticide, des agents antimicrobiens, fongicides ou germicides, des vitamines etc.), un solvant volatile et un polymère filmogène en solution dans ledit solvant, ledit polymère pouvant notamment être choisi parmi les polyéthylène glycol et les propylène glycol et ayant pour effet d'améliorer l'efficacité d'une quantité donnée d'actif.

Toutefois, l'ensemble de ces documents concernent uniquement la co-formulation de ces polymères oxyéthylénés avec un actif.

En outre, rien n'est dit dans ces documents concernant l'utilisation de ces polymères dans des compositions de coloration capillaires contenant des colorants, en particulier des colorants d'oxydation, pour en limiter leur pénétration dans la peau.

Par ailleurs, il est connu de l'homme de l'art d'utiliser des polymères, oxyéthylénés dans des compositions de prétraitement de la peau ou du cuir chevelu, destinées à être appliquées en prétraitement avant l'application d'une composition cosmétique sur la peau ou d'une composition capillaire sur les cheveux.

On peut notamment citer le brevet américain US 6,001,377 décrivant des compositions de soin de la peau et un procédé pour améliorer l'apparence de la peau, lesdites compositions contenant des particules minérales (telles que le dioxyde de titane, l'oxyde de zinc, etc.) et un polymère oxyéthyléné tel qu'un polyéthylène glycol ; et le brevet US 2,449,070 qui décrit un revêtement protecteur pour protéger les mains avant la manucure, à base de dérivé de cellulose, de solvant volatile (tel que l'éthanol), d'émollient et de matériau sous forme de cire tel que le polyéthylène glycol de masse moléculaire moyenne 1000 $g.mol^{-1}$ et le polyéthylène glycol de masse moléculaire moyenne 1500 $g.mol^{-1}$.

En ce qui concerne plus particulièrement les compositions de prétraitement à appliquer sur le cuir chevelu avant un traitement capillaire, on peut citer le brevet US 4,592,908 qui décrit une crème de prétraitement à appliquer avec un processus de défrisage pour protéger le cuir chevelu, contenant de la vaseline, un alcool gras oxyéthyléné en $C_8$-$C_{18}$ ou un alkylphénol oxyéthyléné comprenant de 2 à 30 groupes oxyde d'éthylène, ainsi qu'un acide organique (ou un de ses esters saponifiables) ; le brevet américain US 5,500,218 et la demande internationale WO 93/16678 qui décrivent des compositions capillaires pour éviter la coloration de la peau et la protéger lors d'un processus de coloration des cheveux, contenant 27 à 50 % en poids de polyéthylène glycol ayant un poids moléculaire moyen variant de 2700 à 7500 $g.mol^{-1}$; et enfin le brevet US 4,545,978 qui décrit un procédé de coloration de cheveu impliquant la protection du cuir chevelu, comprenant l'utilisation de substances minérales ayant des propriétés absorbantes et filtrantes, tel que par exemple du charbon actif, de préférence sous forme de suspension dans un polyéthylène glycol.

Aucun de ces documents ne décrit l'utilisation, dans des compositions de prétraitement capillaire à appliquer sur le cuir chevelu ainsi que sur les cheveux avant l'application d'une composition de coloration contenant des colorants, en particulier des colorants d'oxydation, d'un polymère oxyéthyléné choisi parmi les polyéthylène glycol de masse moléculaire moyenne supérieure à 2000 $g.mol^{-1}$ et inférieure à 35000 $g.mol^{-1}$, les polymères séquencés à blocs d'oxyde d'éthylène et d'oxyde de propylène comprenant plus de 75 groupes d'oxyde d'éthylène successifs dans la chaîne par molécule, et le copolymère de polyéthylène glycol comprenant huit groupements d'oxyde d'éthylène successifs et de dicyclohexylméthane diisocyanate.

L'invention a donc pour objet une composition anti-pénétrante de prétraitement capillaire comprenant, dans un milieu physiologiquement acceptable, une quantité efficace supérieure à 5 % et inférieur à 20% en poids par rapport au volume total de la composition d'au moins un polymère oxyéthyléné choisi dans le groupe constitué par :

a) les polyéthylène glycol répondant à la formule générale (1) :

$$H(OCH_2CH_2)_nOH \qquad (1)$$

où n est supérieur à 45 et inférieur à 795,

La quantité de polymère oxyéthyléné dans la composition de l'invention est inférieure à 20 % en poids, de préférence inférieure à 15% en poids, et mieux de l'ordre de 10% en poids par rapport au volume total de la composition.

De manière avantageuse, le polyéthylène glycol a) répondant à la formule (1) comprend 75 à 600 groupes oxydes d'éthylène par molécule.

A titre d'exemples de polyéthylène glycol préférés, on peut citer :

- le polyéthylène glycol PEG-90, comprenant un nombre moyen de groupements éthylène glycol de 90 et ayant une masse moléculaire moyenne théorique Mth d'environ 4000 $g.mol^{-1}$,
- le polyéthylène glycol PEG-100, comprenant un nombre moyen de groupements éthylène glycol de 100 et ayant une masse moléculaire moyenne théorique Mth d'environ 4400 $g.mol^{-1}$,
- le polyéthylène glycol PEG-135, comprenant un nombre moyen de groupements éthylène glycol de 135 et ayant une masse moléculaire moyenne théorique Mth d'environ 6000 $g.mol^{-1}$,
- le polyéthylène glycol PEG-150, comprenant un nombre moyen de groupements éthylène glycol de 150 et ayant

une masse moléculaire moyenne théorique Mth d'environ 6600 $g.mol^{-1}$,

- le polyéthylène glycol PEG-180, comprenant un nombre moyen de groupements éthylène glycol de 180 et ayant une masse moléculaire moyenne théorique Mth d'environ 7900 $g.mol^{-1}$,
- le polyéthylène glycol PEG-200, comprenant un nombre moyen de groupements éthylène glycol de 200 et ayant une masse moléculaire moyenne théorique Mth d'environ 8800 $g.mol^{-1}$,
- le polyéthylène glycol PEG-240, comprenant un nombre moyen de groupements éthylène glycol de 240 et ayant une masse moléculaire moyenne théorique Mth d'environ 10600 $g.mol^{-1}$,
- le polyéthylène glycol PEG-350, comprenant un nombre moyen de groupements éthylène glycol de 350 et ayant une masse moléculaire moyenne théorique Mth d'environ 15400 $g.mol^{-1}$,
- le polyéthylène glycol PEG-454, comprenant un nombre moyen de groupements éthylène glycol de 454 et ayant une masse moléculaire moyenne théorique Mth d'environ 20 000 $g.mol^{-1}$,

la masse moléculaire moyenne théorique Mth d'un polyéthylène glycol étant calculée d'après l'équation (I):

$$Mth = 18 + 44xn \qquad (I)$$

où n est tel que défini précédemment.

A titre d'exemples de PEG-90 disponibles dans le commerce, on peut citer ceux commercialisés sous les dénominations commerciales :

- Lipoxol 4000 MED par la société Condea Chimie,
- Lutrol E-4000 Prill par la société BASF,
- Macrogol 4000 par la société NOF,
- Polyglycol E-4000 par la société DOW CHEMICAL,
- Polyglykol 4000 par la société Clariant GmbH,
- Unipeg-4000 X par la société Universal Preserv-A-Chem.
- Carbowax 4000 par la société Union Carbide
- Polyethylene glycol 4'000 par la Société FLUKA.

A titre d'exemple de PEG-100 disponibles dans le commerce, on peut citer ceux commercialisés sous les dénominations commerciales :

- CARBOWAX PEG 4600 (UNION CARBIDE),
- Polyglycol E-4500 (DOW CHEMICAL).

A titre d'exemple de PEG-135 disponibles dans le commerce, on peut citer ceux commercialisés sous les dénominations commerciales :

- Lipoxol 6000 MED (Condea Chemie)
- Macrogol 6000 (NOF).

A titre d'exemple de PEG-150 disponibles dans le commerce, on peut citer ceux commercialisés sous les dénominations commerciales :

- Lutrol E 6000 Prill (BASF)
- Pluracol E 8000 (BASF)
- Renex PEG 6000 (Uniqema Americas)
- Sabopeg 6000 (Sabo)
- Unipeg-6000 X (Universal Preserv-A-Chem).

A titre d'exemple de PEG-180 disponibles dans le commerce, on peut citer ceux commercialisés sous les dénominations commerciales :

- Calgene PEG 8000 (Calgene)
- CARBOWAX PEG 8000 (UNION CARBIDE)
- Polyglycol E-8000 (DOW CHEMICAL)

- Polyglykol 8000 (Clariant mbH)
- Renex PEG 8000 (UNIQEMA AMERICAS)
- Upiwax 8000 (UNIVERSAL PRESERV-A-Chem).

A titre d'exemple de PEG-200 disponibles dans le commerce, on peut citer ceux commercialisés sous les dénominations commerciales :

- Germinol (Dr. Gerhard steidl)
- Harmonic ASP (Dr. Gerhard Steidl)
- Hexatrate Al-Free (Vevy)
- Jonat AS (Dr. Gerhard Steidl).

A titre d'exemple de PEG-240 disponibles dans le commerce, on peut citer ceux commercialisés sous les dénominations commerciales :

- Lipoxol 12000 (Condea Chemie)
- Polyglykol 12000 (Clariant GmbH).

A titre d'exemple de PEG-350 disponibles dans le commerce, on peut citer ceux commercialisés sous les dénominations commerciales :

- Lipoxol 20000 (Condea Chemie)
- Polyglykol 20000 (Clariant GmbH)
- Upiwax 20000 (Universal Preserv-A-Chem).

A titre d'exemples de PEG-454 disponibles dans le commerce, on peut citer celui commercialisé par la société FLUKA sous la dénomination commerciale Polyéthylène glycol 20'000.

Le milieu physiologiquement acceptable est un milieu liquide ne nuisant pas à l'effet réducteur de l'absorption et du passage transcutané du ou des polymère(s) oxyéthyléné(s) utilisable(s) selon l'invention, ni à la propriété de coloration des colorants, notamment des colorants d'oxydation, contenus dans la composition de coloration capillaire.

Le milieu physiologiquement acceptable est de préférence un milieu solubilisant du (ou des) polymère(s) oxyéthylèné(s) utilisable(s) selon l'invention et à propriété bactériostatique. Il comprend généralement un solvant ou un mélange de solvants du (ou des) polymère(s) oxyéthyléné(s).

Parmi les solvants convenant pour la formulation des compositions selon l'invention, on peut citer l'eau, les alcools et notamment les alcanols inférieurs ($C_1$-$C_6$) tels que l'éthanol et l'isopropanol, l'alcool benzylique et les alcanediols tels que l'éthylèneglycol, le propylène glycol et le pentane diol, les éthers, les esters (en particulier les acétates), le diméthylsulfoxyde (DMSO), la N-méthylpyrrolidone (NMP), les cétones (en particulier l'acétone et leurs mélanges).

Le milieu physiologiquement acceptable comprend de préférence de l'eau, en particulier distillée ou permutée, ou un mélange eau/alcool, en particulier eau/éthanol.

La quantité d'alcool dans le mélange eau/alcool peut représenter jusqu'à 80% en volume, de préférence 70% en volume du mélange eau/alcool.

De manière avantageuse, le mélange eau/alcool est un mélange eau/éthanol, dans lequel l'éthanol représente 70% en volume du mélange eau/éthanol.

Les compositions antipénétrantes de prétraitement capillaire selon l'invention peuvent comprendre tout adjuvant cosmétique conventionnel, en proportion usuelle, qui ne nuit pas aux propriétés recherchées.

A titre d'adjuvant, on peut notamment citer les gélifiants et/ou épaississants classiques, les tensioactifs anioniques, non ioniques, cationiques ou amphotères, les agents propénétrants, les émulsionnants, les parfums, les conservateurs, les charges, les filtres solaires, les protéines, les vitamines, les provitamines, les polymères non fixants anioniques, non ioniques, cationiques ou amphotères, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les bactéricides, les séquestrants, les anti-pelliculaires, les agents alcalinisants, les silicones volatiles ou non, linéaires ou cycliques, modifiées ou non, les polyols, et tout autre additif classiquement utilisé dans les compositions cosmétiques destinées à être appliquées sur les cheveux.

Les quantités de ces différents adjuvants sont celles classiquement utilisées dans le domaine considéré.

Les compositions selon l'invention peuvent être appliquées sur le cuir chevelu, ainsi que sur des cheveux secs ou humides, mais sont de préférence utilisées en mode non rincé, c'est-à-dire que l'on applique les compositions sur le cuir chevelu, ainsi que sur les cheveux (en prétraitement), puis on les laisse poser quelques minutes ou plus avant

de faire la coloration. Mais il est également possible d'utiliser les compositions selon l'invention en mode rincé.

Les compositions selon l'invention peuvent se présenter sous toutes les formes appropriées pour une application topique, notamment sous forme de solutions du type lotion ou sérum ; sous forme de gels aqueux ; sous forme d'émulsions obtenues par dispersion d'une phase grasse dans une phase aqueuse (H/E) ou inversement (E/H), de consistance liquide plus ou moins épaisse telle que des laits et des crèmes plus ou moins onctueuses ; sous forme de spray, ou d'aérosol ; ou encore sous forme de brosse ou de stick.

Ces compositions sont préparées selon les méthodes usuelles.

L'invention a encore pour objet un procédé pour limiter la pénétration dans la peau et/ou les fibres kératiniques d'au moins un colorant, de préférence un colorant d'oxydation, contenu dans une composition de coloration capillaire, caractérisé en ce qu'il consiste à appliquer en prétraitement sur le cuir chevelu et sur les cheveux, avant un processus de coloration des cheveux utilisant la composition de coloration capillaire, une composition anti-pénétrante de prétraitement capillaire selon l'invention.

Généralement, la durée d'application de la composition anti-pénétrante selon l'invention sur le cuir chevelu et sur les cheveux varie de cinq secondes à une heure, de préférence de 1 à 10 minutes.

Généralement, les colorants d'oxydation sont tous des composés ou mélanges de composés qui, en présence d'un oxydant, par exemple l'oxygène de l'air ou l'eau oxygénée, s'oxydent pour donner un composé ou un mélange de composés colorés.

Les colorants d'oxydation sont généralement choisis parmi les bases d'oxydation, les coupleurs, les orthodiphénols et leurs mélanges.

Les bases d'oxydation, de type para ou ortho, sont des composés qui ne sont pas des colorants en eux-mêmes, mais forment un colorant par un processus de condensation oxydative, soit sur eux-mêmes, soit en présence d'un coupleur ou modificateur. Ils comportent des groupements fonctionnels, soit deux groupements amino, soit un groupement amino et un groupement hydroxy en position para ou ortho l'un par rapport à l'autre.

La nature de ces bases d'oxydation n'est pas critique. Elles peuvent notamment être choisies parmi les ortho- et para-phénylènediamines, les bases doubles, les ortho- et para-aminophénols, les bases hétérocycliques ainsi que les sels d'addition de tous ces composés avec un acide.

A titre de paraphénylènediamines, on peut notamment citer les paraphénylènediamines de formule chimique (4) suivante et leurs sels d'addition avec un acide :

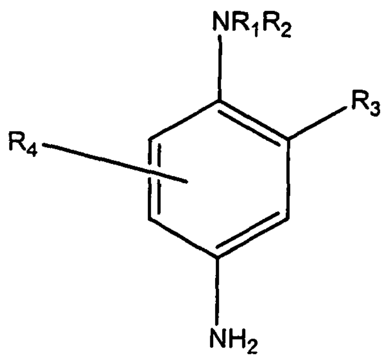

(4)

dans laquelle :

- $R_1$ représente un atome d'hydrogène, un radical alkyle en $C_1$-$C_4$, monohydroxyalkyle en $C_1$-$C_4$, polyhydroxyalkyle en $C_2$-$C_4$ alcoxy($C_1$-$C_4$)alkyle($C_1$-$C_4$), alkyle en $C_1$-$C_4$ substitué par un groupement azoté, phényle ou 4'-aminophényle ;
- $R_2$ représente un atome d'hydrogène, un radical alkyle en $C_1$-$C_4$, monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_3$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$ ;,
- $R_4$ représente un atome d'hydrogène, d'halogène ou un radical alkyle en $C_1$-$C_4$ ;
- $R_1$ et $R_2$ peuvent également former avec l'atome d'azote qui les porte un hétérocycle azoté à 5 ou 6 chaînons éventuellement substitué par un ou plusieurs groupements alkyle, hydroxy ou uréido.

Parmi les groupements azotés de la formule (4) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les paraphénylènediamines de formule (4) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl) amino-2-méthyl-aniline, la 4-N,N-bis-(β-hydroxyéthyl)-amino 2-chloro-aniline, la 2-β-hydroxyéthylparaphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthylparaphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxyparaphénylènediamine, la N-(β-méthoxyéthyl)-paraphénylènediamine, 2-méthyl-1-N-β-hydroxyéthyl-paraphénylènediamine, et leurs sels d'addition avec un acide.

Parmi les paraphénylènediamines de formule (4) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthylparaphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (5) suivante, et leurs sels d'addition avec un acide :

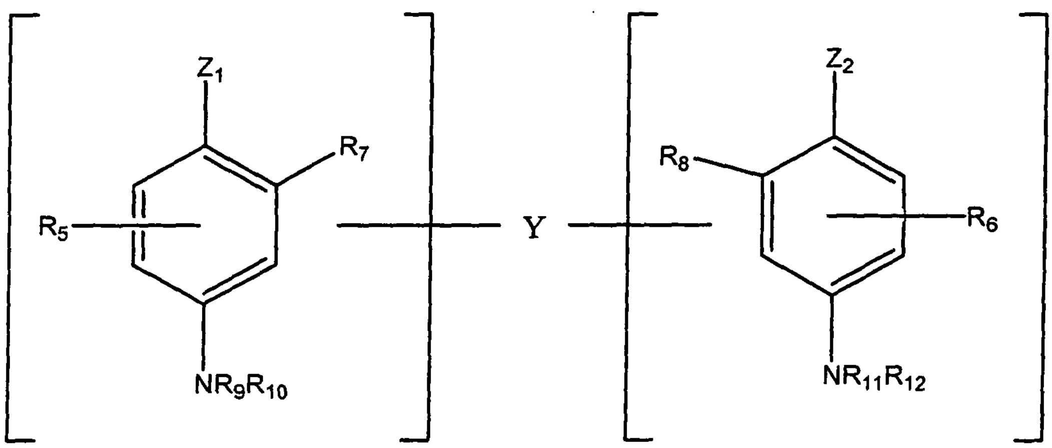

(5)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_7$, $R_8$, $R_9$, $R_{10}$, $R_{11}$ et $R_{12}$, identiques ou différents, représentent un atome d'hydrogène, un bras de liaison Y ou un radical alkyle en $C_1$-$C_4$ ;

étant entendu que les composés de formule (5) ne comportent qu'un seul bras de liaison Y par molécule.

Parmi les groupements azotés de la formule (5) ci-dessus, on peut citer notamment les radicaux amino, mo-

noalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les bases doubles de formules (5) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (5), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols, on peut notamment citer les para-aminophénols répondant à la formule (6) suivante, et leurs sels d'addition avec un acide :

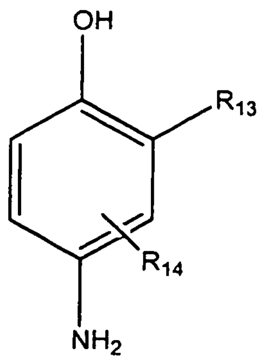

(6)

dans laquelle :

- $R_{13}$ représente un atome d'hydrogène, un atome d'halogène tel que le fluor, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl($C_1$-$C_4$)aminoalkyle en $C_1$-$C_4$, et
- $R_{14}$ représente un atome d'hydrogène ou un atome d'halogène tel que le fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$) alkyle($C_1$-$C_4$).
  Parmi les para-aminophénols de formule (6) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

Les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB-1026978 et GB-1153196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE-2359399 ou japonais JP 88-169571 et JP-9110659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxyéthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5,N7,N7-tetraméthyl-pyrazolo-[1,5-a]-pyrimidine-3,7-diamine; la 3-amino-5-méthyl-7-imidazolylpropylamino pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE-3843892, DE-4133957 et demandes de brevet WO 94/08969, WO 94/08970, FR-A-2 733 749 et DE-19543988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl pyrazole, le 4,5-diamino 1-méthyl-3-phénylpyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)- pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropylpyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Une autre classe de colorants d'oxydation est constituée par les orthodiphénols. Ce sont des composés comportant au moins un cycle aromatique dont au moins deux carbones consécutifs portent un groupe hydroxyle. De préférence, le cycle aromatique est un cycle benzénique ou un cycle aromatique condensé.

Le cycle aromatique peut être un cycle aromatique condensé contenant éventuellement un ou plusieurs hetéroatomes, tel que le naphtalène, le tétrahydronaphtalène, l'indane, l'indène, l'anthracène, le phénanthrène, l'indole, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine.

Les colorants d'oxydation orthodiphénols préférés peuvent être représentés par la formule (7) :

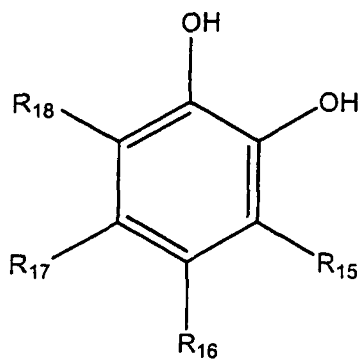

(7)

dans laquelle les substituants $R_{15}$ à $R_{18}$, identiques ou différents, représentent un atome d'hydrogène, un radical halogène, hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvant être éventuellement substitué, aryle, aryle substitué, un radical hétérocyclique éventuellement substitué, un radical contenant un ou plusieurs atomes de silicium, où deux des substituants $R_{15}$ à $R_{18}$ forment conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

Les cycles saturés ou insaturés, éventuellement condensés, peuvent être aussi éventuellement substitués.

Les radicaux alkyles sont généralement les radicaux alkyles en $C_2$-$C_{10}$, de préférence les radicaux alkyles en $C_1$-$C_6$, tels que méthyle, éthyle, propyle, butyle, pentyle et hexyle.

Les radicaux alcoxy sont en général les radicaux alcoxy en $C_1$-$C_{20}$, tels que méthoxy, éthoxy, propoxy et butoxy.

Les radicaux alcoxy alkyles sont de préférence les radicaux alcoxy ($C_1$-$C_{20}$) alkyle ($C_1$-$C_{20}$), tels que méthoxyméthyle, éthoxyméthyle, méthoxyéthyle, éthoxyéthyle, etc.

Les radicaux cycloalkyles sont en général les radicaux cycloalkyles en $C_4$-$C_8$, de préférence les radicaux cyclopentyle et cyclohexyle. Les radicaux cycloalkyles peuvent être des radicaux cycloalkyles substitués, en particulier par des groupes alkyles, alcoxy, acide carboxylique, hydroxyle, amine et cétone.

Les radicaux alcényles sont de préférence des radicaux en $C_2$-$C_{20}$, tels que éthylène, propylène, butylène, pentylène, méthyl-2-propylène et décylène.

Les radicaux contenant un ou plusieurs atomes de silicium sont de préférence des radicaux polydiméthylsiloxane, polydiphenylsiloxane, polydiméthylphénylsiloxane, stéraoxydiméthicone.

Les radicaux hétérocycliques sont en général des radicaux comprenant un ou plusieurs hétéroatomes choisis parmi O, N et S, de préférence O ou N, éventuellement substitués par un ou plusieurs groupes alkyles, alcoxy, acide carboxylique, hydroxyle, amine ou cétone.

Parmi les radicaux hétérocyliques préférés, on peut citer les groupes furyle, pyrannyle, pyrrolyle, imidazolyle, pyrazolyle, pyridyle, thiényle.

De préférence encore, les groupes hétérocycliques sont des groupes condensés tels que des groupes benzofurannyle, chromènyle, xanthényle, indolyle, isoindolyle, quinolyle, isoquinolyle, chromannyle, isochromannyle, indolinyle, isoindolinyle, coumarinyle, isocoumarinyle, ces groupes pouvant être substitués, en particulier par un ou plusieurs groupes OH. Les colorants orthodiphénols préférés sont :

- les flavanols comme la catéchine et le gallate d'épichatéchine,
- les flavonols comme la quercétine,
- les anthocyanidines comme la péonidine,
- les anthocyanines, par exemple l'oenine,
- les hydroxybenzoates, par exemple l'acide gallique,
- les flavones comme la lutéoline,
- les iridoïdes comme l'oleuropéine,

ces produits pouvant être osylés (par exemple glucosylés) et /ou sous forme d'oligomères (procyanidines) ;

- les hydroxystilbènes, par exemple le tétrahydroxy-3,3',4,5'-stilbène, éventuellement osylés (par exemple glucosylés) ;
- la 3,4-dihydroxyphénylalanine et ses dérivés ;
- la 2,3-dihydroxyphénylalanine et ses dérivés ;
- la 4,5-dihydroxyphénylalanine et ses dérivés ;
- le 4,5-dihydroxyindole et ses dérivés ;
- le 5,6-dihydroxyindole et ses dérivés ;
- le 6,7-dihydroxyindole et ses dérivés ;
- le 2,3-dihydroxyindole et ses dérivés ;
- les dihydroxycinnamates tels que l'acide caféique et l'acide chlorogénique ;
- les hydroxycoumarines ;
- les hydroxyisocoumarines ;
- les hydroxycoumarones;
- les hydroxyisocoumarones;
- les hydroxychalcones ;
- les hydroxychromones ;
- les anthocyanes ;
- les quinones ;
- les hydroxyxanthones ; et
- les mélanges de ceux-ci.

Lorsque les colorants présentent des formes D et L, les deux formes peuvent être utilisées dans les compositions selon l'invention.

Les orthodiphénols particulièrement préférés sont le 5,6-dihydroxyindole et l'acide 5,6-dihydroxyindole carboxylique.

Les orthodiphénols tels que définis précédemment peuvent être contenus dans des extraits de plantes, fruits, agrumes, légumes et des mélanges de ces extraits.

Parmi les extraits de plantes, on peut citer les extraits de rose et de thé.

Parmi les extraits de fruits, on peut citer les extraits de pomme, de raisin (en particulier de pépins de raisin) et

de banane.

Parmi les extraits de légumes, on peut citer l'extrait de pomme de terre.

On peut également utiliser des mélanges d'extraits de plantes et/ou de fruits tels que des mélanges d'extraits de pomme et de thé et des mélanges d'extraits de raisin et de pomme.

Suivant les parties de fruits utilisés, par exemple pulpe ou pépins de raisin, la coloration obtenue est différente.

Les orthodiphénols peuvent être utilisés conjointement avec les bases d'oxydation.

Une autre classe de colorants d'oxydation est constituée par les coupleurs.

Les coupleurs peuvent être choisis parmi ceux classiquement utilisés en teinture d'oxydation et notamment parmi les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide, ces composés étant différents des composés orthodihydroxylés de l'invention.

Ces coupleurs sont plus particulièrement choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Généralement le ou les coupleurs représentent de préférence de 0,0001 à 15% en poids environ du poids total de la composition tinctoriale prête à l'emploi et encore plus préférentiellement de 0,001 à 10% environ.

Les bases d'oxydation et les coupleurs constituent des colorants d'oxydation. Les sels d'addition avec un acide de ces colorants d'oxydation sont notamment choisis parmi les chlorhydrates, les bromhydrates, les sulfates et les tartrates, les lactates et les acétates.

La quantité de colorants d'oxydation dans la composition de coloration doit être suffisante pour obtenir une coloration visible. Cette quantité peut varier dans de larges mesures en fonction de la nature du colorant d'oxydation et de l'intensité voulue pour la coloration.

En général, on obtiendra une coloration convenable lorsque la quantité de colorant est telle que la teneur en colorant dans la composition de coloration finale est d'au moins 0,1 micromole, de préférence d'au moins 1 micromole par millilitre de composition finale.

Typiquement, la quantité de colorant d'oxydation dans la composition de coloration varie de 1 mM à 10 mM par litre et généralement de l'ordre de 5 mM par litre.

En faisant varier la nature des différents colorants d'oxydation et leurs proportions dans la composition, on peut faire varier la couleur de la composition de coloration finale. On obtient ainsi une palette de couleurs.

Les compositions de coloration capillaire peuvent, outre les colorants d'oxydation, renfermer un ou plusieurs colorants directs, notamment pour modifier les nuances en les enrichissant de reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

Les compositions de coloration capillaire peuvent également contenir une quantité effective d'au moins un acide aminé en particulier comportant au moins un groupe thiol (SH) et de préférence un seul groupe thiol, ces acides aminés pouvant être sous la forme de chlorhydrates et/ou au moins une protéine, en particulier un peptide.

Les acides aminés préférés selon l'invention sont les acides aminés qui contiennent une fonction amine en position α par rapport à une fonction acide carboxylique.

Les acides aminés préférés peuvent être représentés par la formule

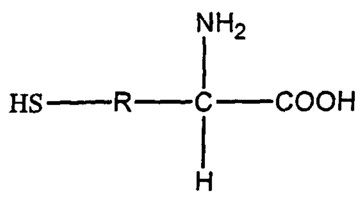

(8)

dans laquelle -R est un radical hydrocarboné divalent, linéaire ou ramifié, par exemple en $C_1$-$C_{10}$, de préférence en $C_1$-$C_6$, tel qu'un radical méthylène, éthylène, butylène, éthylidène, propylidène, un radical cyclique saturé divalent, éventuellement substitué, par exemple en $C_4$-$C_8$, un groupe aromatique divalent, éventuellement substitué, tel qu'un radical phénylène, tolylène, xylylène.

Parmi les acides aminés préférés pour les compositions de l'invention, on peut citer la cystéine et ses dérivés, en particulier la L-cystéine et le chlorhydrate de L-cystéine.

Parmi les protéines, on peut citer le gluthation et ses dérivés et la protéine de soja.

Les proportions relatives d'acide aminé et/ou de protéine et de colorant d'oxydation dans les compositions de l'invention peuvent varier dans de larges mesures en fonction de la coloration voulue. Généralement, le rapport molaire acide aminé/colorant d'oxydation variera de 0,001 à 50, de préférence de 0,01 à 5, et mieux de 0,05 à 2,5.

En général, la teneur en acide aminé à groupe thiol dans la composition finale est d'au moins 0,01 micromole par millilitre, de préférence au moins 0,1 micromole/ml.

En faisant varier la nature des précurseurs de colorant et des acides aminés de la composition et la proportion relative d'acide aminé et de précurseur de colorant, on peut obtenir toute une palette de teintes.

Les compositions selon l'invention peuvent en outre comprendre une ou plusieurs enzymes.

L'enzyme ou les enzymes présentes dans les compositions selon l'invention peuvent être toutes enzymes présentant une activité propigmentation.

L'activité propigmentation peut se définir comme l'activité enzymatique qui catalyse l'oxydation d'un substrat pour conduire à la formation de pigments.

Les enzymes peuvent notamment être choisis parmi les pyranose oxydases, les glucose oxydases, les glycérol oxydases, les lactate oxydases, les pyruvate oxydases, les uricases, les choline oxydases, les sarcosine oxydases, les bilirubines oxydases, les laccases, les tyrosinases, les péroxydases, les catalases, les superoxydesdimutases et leurs mélanges, ou parmi des extraits végétaux ou animaux contenant des enzymes précitées, en présence éventuelle d'un donneur (ou substrat) nécessaire au fonctionnement desdites enzymes tel que par exemple la L-tyrosine ou la L-DOPA.

Les enzymes utilisées selon l'invention peuvent être d'origine animale, microbiologique (bactérienne, fongique ou virale) ou synthétique (obtenue par synthèse chimique ou biotechnologique).

La ou les enzymes peuvent être utilisées sous forme cristalline pure ou sous une forme diluée dans un diluant inerte pour ladite enzyme.

A titre d'exemple d'uricases, on peut notamment citer l'uricase extraite de foie de sanglier, l'uricase d'Arthrobacter globiformis, ainsi que l'uricase d'Aspergillus flavus.

A titre d'exemple de sources de choline oxydase, on peut notamment citer le foie de rat, les bactéries telles que Arthrobacter globiformis, Achromobacter cholinophagum ou Alcaligenes, et les champignons tels que Cylindrocarpon didynum.

A titre d'exemple de sources de sarcosine oxydase, on peut notamment citer les bactéries telles que Arthrobacter et en particulier Arthrobacter ureafaciens et Arthrobacter globiformis, Streptomyces, Bacillus, Pseudomonas, Corynebacterium ou Alcaligenes tels que par exemple Alcaligenes denitrificans, et les champignons tels que Cylindrocarpon didynum.

A titre d'exemple de sources de bilirubine oxydase, on peut notamment citer la muqueuse intestinale et le foie de rat, les bactéries telles que Myrothecium verucania, Myrothecium cinctum, et Myrothecium roridum.

Parmi les laccases d'origine végétale utilisables selon l'invention, on peut citer les laccases produites par des végétaux effectuant la synthèse chlorophylienne telles que celles indiquées dans la demande de brevet FR-A-2.694.018.

On peut notamment citer les laccases extraites d'Anacardiacées, de Podocarpacées, de Rosmarinus off., de Solanum tuberosum, dlris sp., de Coffea sp., de Daucus carrota, de Vinca minor, de Persea americana, de Catharenthus roseus, de Musa sp., de Malus pumila, de Gingko biloba, et de Monotropa hypopithys (sucepin).

Parmi les laccases d'origine microbienne (notamment fongique), ou obtenues par biotechnologie utilisables selon l'invention, on peut citer les laccases de Polyporus versicolor, de Rhizoctonia praticola et de rhus vernicifera telles que décrites par exemple dans les demande de brevet FR-A-2.112.549 et EP-A-504.005 ; les lacases décrites dans les demandes de brevet WO95/07988, WO95/33836, WO95/33837, WO96/00290, WO97/19998 et WO97/19999 dont le contenu fait partie intégrante de la présente description comme par exemple les laccases de Scytalidium, de Polyporus pinsitus, de Myceliophthora thermophila, de Rhizoctonia solani, de Pyricularia orizae, et leurs variantes.

On choisira plus préférentiellement les laccases d'origine microbienne ou celles obtenues par biotechnologie.

Dans une forme de réalisation particulièrement préférée de l'invention, l'enzyme utilisée correspond à la tyrosinase (nomenclature EC 1.14.18.1). Par tyrosinase, il faut entendre dans la présente invention toute enzyme présentant une activité tyrosinase, cette enzyme pouvant présenter d'autres activités enzymatiques. L'activité tyrisonase peut se définir comme l'activité enzymatique qui catalyse l'oxydation de la tyrosine pour conduire à la formation du précurseur de mélanine : la Dopaquinone.

A titre d'exemple de sources de tyrosinase, on peut notamment citer la pomme de terre, les champignons, les micro-organismes tels que Neurospora crassa, etc.

La quantité d'enzyme présente dans la composition finale peut varier largement mais est généralement de $5.10^{-3}$ à 5 mg, de préférence $5.10^{-2}$ à 0,5 mg par millilitre de composition finale.

Les compositions de coloration peuvent également contenir d'autres adjuvants cosmétiquement acceptables, tels que par exemple des agents tensioactifs, des agents épaississants, des agents de pénétration, des parfums, des tampons, et divers adjuvants usuels comme des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, des agents réducteurs, des émulsionnants, des conservateurs, des charges, des filtres solaires, des protéines, des polymères fixants anioniques, non ioniques, cationiques ou amphotères, des agents hydratants, des émollients, des agents adoucissants, des agents anti-mousse, des agents antiperspirants, des agents anti-radicaux libres, des polymères fixants ou non, des bactéricides, des séquestrants, des anti-pelliculaires, des anti-oxydants, des agents alcalinisants, des polyols, et tout autre additif classiquement utilisé dans les compositions cosmétiques destinées à être appliquées sur les cheveux.

Les agents tensio-actifs peuvent être choisis parmi les agents tensio-actifs anioniques, cationiques, non-ioniques, amphotères ou leurs mélanges, et de préférence parmi les tensio-actifs non ioniques.

Parmi ces agents tensio-actifs, on peut citer les alkylbenzènesulfonates, les alkylnaphtalènesulfonates, les sulfates, les éthers sulfates et les sulfonates d'alcools gras, les sels d'ammonium quaternaires, tels que le bromure de triméthylcétylammonium, le bromure de cétylpyridinium; les éthanolamides d'acides gras éventuellement oxyéthylénés; les acides, les alcools ou les amines polyoxyéthylénés, les alcools polyglycérolés, les alkylphénols polyoxyéthylénés ou polyglycérolés, ainsi que les alkylsulfates polyoxyéthylénés.

Les agents épaississants que l'on peut ajouter dans les compositions de coloration peuvent être choisis parmi l'alginate de sodium, la gomme arabique, les dérivés de cellulose, les polymères d'acide acrylique, la gomme de xanthane. On peut également utiliser les agents épaississants minéraux tels que la bentonite.

Les filtres UV organiques peuvent être notamment choisis parmi les dérivés cinnamiques ; les dérivés de dibenzoylméthane ; les dérivés salicyliques, les dérivés du camphre ; les dérivés de triazine tels que ceux décrits dans les demandes de brevet US 4,367,390, EP-0.863.145, EP-0.517.104, EP-0.570.838, EP-0.796.851, EP-0.775.698, EP-0.878.469 , EP-0.933.376 et EP-0.893.119 ; les dérivés de la benzophénone ; les dérivés de β,β'-diphénylacrylate, les dérivés de benzimidazole ; les dérivés bis-benzoazolyle tels que ceux décrits dans les brevets EP-0.669.323 et US 2,463,264 ; les dérivés de méthylène bis-(hydroxyphénylbenzotriazole) tels que ceux décrits dans les demandes US 5,237,071, US 5,166,355, GB-2303549, DE-19726184 et EP-0.893.119 ; les dérivés de l'acide p-aminobenzoïque ; les dimères dérivés d'α-alkylstyrène tels que décrits dans la demande de brevet DE-19855649 ; les polymères hydrocarbonés filtres et les silicones filtres tels que ceux décrits notamment dans la demande WO 93/04665. On peut aussi utiliser des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-0.518.772 et EP-0.518.773.

Les compositions de coloration utilisables selon l'invention peuvent également comprendre une quantité effective d'au moins un colorant d'oxydation et une quantité effective d'un système limitant le passage transcutané du colorant d'oxydation comprenant un premier constituant choisi parmi les sels et oxydes de Mn(II) et/ou Zn(II) et un second constituant choisi parmi les hydrogénocarbonates alcalins, les hydrogénocarbonates alcalino-terreux et leurs mélanges, les proportions du premier constituant et du second constituant étant telles que :

$$\frac{[Mn(II)]}{[HCO_3]} \leq 1 \text{ avec } [\mathrm{Mn(II)}] \neq 0$$

$$\frac{[Zn(II)]}{[HCO_3]} \leq 1 \text{ avec } [\mathrm{Zn(II)}] \neq 0$$

$$\frac{[Mn(II) + Zn(II)]}{[HCO_3]} \leq 1 \text{ avec } [\text{Mn(II)}] \text{ et } [\text{Zn(II)}] \neq 0$$

où [Mn(II)], [Zn(II)] et [$HCO_3$] représentent respectivement les concentrations molaires en Mn(II), Zn(II) et $HCO_3$ dans la composition.

Généralement, le rapport $\frac{[Mn(II)]}{[HCO_3]}$ varie de $10^{-5}$ à $10^{-1}$, de préférence de $10^{-3}$ à $10^{-2}$ et mieux est de l'ordre de $5.10^{-3}$.

Dans le cas de Zn(II), le rapport $\frac{[Zn(II)]}{[HCO_3]}$ est en général d'un ordre de 10 à 100 fois supérieur au rapport dans le cas Mn(II).

Typiquement, ce rapport est de $10^{-4}$ ou plus, de préférence $10^{-3}$ ou plus, et de préférence de l'ordre de $5.10^{-1}$.

Dans le cas d'un mélange de Mn(II) et Zn(II), le rapport varie généralement de $10^{-5}$ à $10^{-1}$, de préférence $10^{-3}$ à $10^{-2}$, ce rapport étant choisi plus élevé lorsque la proportion de Zn(II) dans le mélange s'accroît.

Généralement, la concentration molaire en Mn(II), Zn(II), ou Mn(II) + Zn(II) dans la composition finale varie de $10^{-3}$ à 10 mM/l, de préférence de $10^{-2}$ à 1 mM/l.

Lorsqu'on utilise seulement un ou plusieurs sels ou oxydes de Mn(II), la concentration molaire en Mn(II) dans la composition finale est typiquement de $10^{-3}$ à $10^{-1}$ mM/l, de préférence $10^{-2}$ à $10^{-1}$ mM/l.

De préférence, lorsqu'on utilise uniquement un ou plusieurs sels ou oxydes de Zn(II), la concentration en Zn (II) dans la composition finale est de $5.10^{-2}$ à 10 mM/l, mieux de $5.10^{-1}$ à 1 mM/l.

Parmi les sels de Mn(II) et Zn(II) convenant pour la présente invention, on peut citer les chlorure, fluorure, iodure, sulfate, phosphate, nitrate et perchlorate, les sels d'acides carboxyliques et leurs mélanges.

A titre d'exemple, on peut citer le chlorure de manganèse, le carbonate de manganèse (par exemple rhodochrosite), le difluorure de Mn(II), l'acétate de Mn(II) tétra hydraté, le lactate de Mn(II) tri hydraté, le phosphate de Mn(II), l'iodure de Mn(II), le nitrate de Mn(II) tri hydraté, le bromure de Mn(II) et le perchlorate de Mn(II) tétra hydraté, et le sulfate de Mn(II) mono hydraté.

Les sels particulièrement préférés sont $MnCl_2$ et $ZnCl_2$ et tout particulièrement $MnCl_2$.

Les sels d'acides carboxyliques incluent également des sels d'acides carboxyliques hydroxylés tels que le gluconate.

Parmi les hydrogénocarbonates alcalins et alcalino-terreux, on peut citer les hydrogénocarbonates de Na, K, Mg, Ca et leurs mélanges, préférentiellement l'hydrogénocarbonate de Na.

De préférence, les compositions selon l'invention sont exemptes d'agents de chélation des sels de Mn(II) et/ou Zn(II) utilisés, car ces agents tendent à inhiber l'oxydation des colorants.

Les exemples suivants illustrent la présente invention.

<u>EXEMPLES</u>

On a réalisé des essais de coloration de soies d'oreille de porc, sur lesquelles on a appliqué en prétraitement (sous condition d'usage) différentes compositions contenant chacune au moins un polymère oxyéthyléné et/ou oxypropyléné, puis une teinture à base de para-phénylène diamine (PPD).

Ces essais ont été réalisés *ex vivo* sur une oreille de porc (d'origine abattoir), car celle-ci représente un modèle de peau proche du cuir chevelu et possède en plus des soies qui représentent les cheveux.

Les oreilles de porc ont été montées dans des cellules de diffusion en mode statique. Avant montage de la peau, les soies ont été coupées à une longueur de 5mm.

La PPD a été formulée dans un support commercialisé par L'OREAL sous la dénomination Récital®. Une teinture composée d'un mélange Récital® contenant la PPD et 20 volumes d'eau oxygénée à un ratio de volume 1 :1 a été préparée juste avant l'application.

Ces essais ont pour but d'évaluer la capacité des polymères oxyéthylénés et/ou oxypropylénés à limiter le passage transcutané de la PPD sans interférer avec le processus de coloration des soies.

A cet effet, on analyse donc, après application successive de la composition de prétraitement et de la teinture sur les cheveux et la peau, le taux d'absorption cutanée de la PPD, ainsi que le taux de PPD dans les soies (comme indice de l'effet du prétraitement sur le processus de coloration des soies).

Pour faciliter l'analyse, on utilise la PPD radiomarquée [$^{14}C$]-PPD.

Les polymères suivants ont été testés seuls ou en mélange :

- Polymères utilisables selon l'invention :
  - PEG 90 commercialisé par la société Fluka sous la dénomination commerciale Polyethylene glycol 4'000 de masse moléculaire moyenne variant de 3500 à 4500 g.mol$^{-1}$.
  - PEG 20000 commercialisé par la société Fluka sous la dénomination commerciale Polyethylene glycol 20'000 de masse moléculaire moyenne variant de 16000 à 24000 g.mol$^{-1}$,
- Polymères testés à titre comparatif :
  - PEG-6 commercialisé par la société Fluka sous la dénomination commerciale Polyethylene glycol 300, de masse moléculaire moyenne variant de 285 à 315 g.mol$^{-1}$,
  - PEG-45 commercialisé par la société Aldrich sous la dénomination commerciale Polyethylene glycol 2'000, de masse moléculaire moyenne variant de 1900 à 2200 g.mol$^{-1}$,
  - PEG-795 commercialisé par la société Fluka sous la dénomination commerciale Polyethylene glycol 35'000, de masse moléculaire moyenne d'environ 35000 g.mol$^{-1}$,
  - Poloxamer 188 (nom CTFA) commercialisé par la société BASF sous la dénomination commerciale Lutrol F-68®,
  - Copolymère de polypropylène glycol comprenant douze groupements d'oxyde d'éthylène successifs et de dicyclohexylméthane diisocyanate distribué en France par la société Sederma, sous la dénomination commerciale Polyolprépolymer-2® (ou PP-2®).

<u>Réalisation des essais</u>

On réalise des compositions de prétraitement capillaire à base d'au moins un polymère oxyéthyléné et/ou oxypropyléné en solubilisant le (ou les) polymère(s) dans de l'éthanol (70% vol).

On applique alors sur les oreilles de porc, les compositions de prétraitement à raison de 20 mg/cm$^2$ pendant 10 minutes. Après séchage (un " film " polymérique s'étant alors formé), la teinture capillaire contenant de la [$^{14}C$]-PPD formulée dans le support Récital® est ensuite appliquée pendant 30 minutes, suivie d'un lavage intensif simulant le shampooing après coloration.

Après une période 24 heures suivant l'application de la teinture, on analyse le taux de [$^{14}C$]-PPD et/ou de [$^{14}C$]-dérivés dans le liquide récepteur ou dans la peau incluant les soies (5 soies, totalité des soies).

L'application de la teinture sans prétraitement a servi de contrôle.

On évalue alors les résultats du prétraitement capillaire en comparaison avec les résultats obtenus sans prétraitement, par la quantification de deux effets :

- l'effet anti-pénétration, qui évalue le taux d'absorption cutanée de la PPD et de ses dérivés, et
- l'effet soies, qui évalue le taux de pénétration de la PPD et de ses dérivés dans les soies.

L'effet soies est un indicateur de l'influence du prétraitement sur la pénétration d'un colorant d'oxydation dans les soies (ou les cheveux). On recherche donc un agent anti-pénétrant qui n'influence pas la pénétration d'un colorant dans les soies (ou les cheveux), c'est-à-dire qui n'interagit pas avec le processus de la coloration.

Il est donc intéressant que l'application de la composition de prétraitement capillaire avant le processus de coloration ait un effet anti-pénétration sans présenter d'effet soies.

L'effet soies, exprimé en pourcentage, correspond à la différence entre les quantités de [$^{14}C$]-PPD et/ou de [$^{14}C$]-dérivés retrouvées dans 5 soies après une coloration réalisée sans prétraitement (contrôle), et celles retrouvées après le même processus de coloration mais réalisé avec prétraitement.

Pour les deux effets (antipénétration et soies), un pourcentage négatif démontre une diminution du taux retrouvé dans le compartiment analysé, tandis qu'un pourcentage positif démontre une augmentation de ce taux.

L'effet anti-pénétration est considéré comme significatif, si, après un prétraitement, la quantité de [$^{14}C$]-PPD et/ou de [$^{14}C$]-dérivés retrouvée dans l'ensemble formé par le stratum corneum , l'épiderme malpighien, le derme et le liquide récepteur (ci-après désigné par « peau totale + LR ») a diminué de plus de 25 % par rapport au même processus de coloration sans prétraitement. L'effet antipénétration est considéré comme important si la quantité de [$^{14}C$]-PPD et/ou de [$^{14}C$]-dérivés dans l'ensemble " peau totale + LR " a diminué de plus de 50 % par rapport à la peau non traitée (contrôle).

Ainsi, on indiquera :

- par le signe " 0 ", les essais ne montrant pas d'effet antipénétration significatif,

- par le signe " - ", les essais montrant un effet pro-pénétrant, c'est-à-dire les essais pour lesquels le prétraitement a augmenté la quantité de [$^{14}$C]- PPD et/ou de [$^{14}$C]-dérivés retrouvée dans l'ensemble « peau totale + LR »,
- par le signe " + ", les essais montrant un effet antipénétration significatif, et
- par le signe "++", ceux montrant un effet antipénétration important.

Le prétraitement est considéré comme non interférant avec le processus de la coloration, si la quantité de [$^{14}$C]-PPD et/ou de [$^{14}$C]-dérivés retrouvée dans 5 soies n'a pas été changée de $\pm$ 25 % par rapport au même processus de coloration sans prétraitement. L'effet soies est considéré comme significatif si, après traitement, la quantité de [$^{14}$C]-PPD et/ou de [$^{14}$C]-dérivés retrouvée dans 5 soies a augmenté ou diminué de plus de 25 % par rapport au même processus de coloration sans prétraitement. L'effet soies est considéré comme important si, après traitement, la quantité de de [$^{14}$C]-PPD et/ou de [$^{14}$C]-dérivés retrouvée dans 5 soies a augmenté ou diminué de plus de 50 % par rapport au même processus de coloration sans prétraitement.

Le prétraitement est considéré comme empêchant la coloration de la soie, si, après traitement, la quantité de [$^{14}$C]-PPD et/ou de [$^{14}$C]-dérivés retrouvée dans 5 soies a été diminué de plus de 25% par rapport au même processus de coloration sans prétraitement. On indiquera :

- par le signe " 0 ", les essais ne montrant pas d'effet soies,
- par le signe " - ", les essais montrant un effet soies empêchant la coloration,
- par le signe " + ", les essais montrant un effet soies significatif, et
- par le signe " ++ ", les essais montrant un effet soies important.

EXEMPLE COMPARATIF

A titre comparatif, on a réalisé un essai de coloration de soies d'oreilles de porc, sur lesquelles on a appliqué en prétraitement avant coloration, une solution d'éthanol (70% en vol.).

Les résultats (évaluation des effets antipénétration et soies) de cet essai sont présentés dans le tableau 1 ci-après :

TABLEAU 1

| | Effet anti-pénétration | Effet soies |
|---|---|---|
| Solution d'éthanol à 70% vol. (véhicule) | - | + |

Les résultats de ces essais montrent qu'une solution d'éthanol à 70% vol. conduit à une augmentation de la quantité [$^{14}$C]-PPD et/ou [$^{14}$C]-dérivés retrouvée dans l'ensemble «peau totale + LR » et à l'apparition d'un effet soies significatif.

EXEMPLE 1

On a réalisé des essais de coloration de soies d'oreilles de porc sur lesquelles on a appliqué, en prétraitement avant coloration, une solution alcoolique contenant 10% en poids d'un polyéthylène glycol par rapport au volume total de la composition. Les résultats (évaluation des effets anti-pénétration et soies) de ces essais sont présentés dans le tableau 2 ci-après.

TABLEAU 2

| Solution contenant 10 % en poids* de polyéthylène glycol** | Masse moléculaire théorique *** (g.mol$^{-1}$) | Masse moléculaire d'après le fournisseur (g.mol$^{-1}$) | n** | Effet anti-pénétration | Effet soies |
|---|---|---|---|---|---|
| PEG-6 | 300 | 285-315 | 6,4 | 0 | 0 |
| PEG-45 | 2000 | 1900-2200 | 45 | 0 | 0 |
| PEG-90 | 4000 | 3500-4500 | 90,5 | + | 0 |
| PEG-454 | 20 000 | 16000-24000 | 454,1 | + | 0 |

(suite)

| Solution contenant 10 % en poids* de polyéthylène glycol** | Masse moléculaire théorique *** ($g.mol^{-1}$) | Masse moléculaire d'après le fournisseur ($g.mol^{-1}$) | n** | Effet anti-pénétration | Effet soies |
|---|---|---|---|---|---|
| PEG-795 | 35000 | ~35000 | 795 | 0 | ++ |

*les pourcentages sont exprimés en poids par rapport au volume total de la composition.
** nombre de groupements oxydes d'éthylène dans un polyéthylène glycol répondant à la formule (1) :

$$H[(OCH_2CH_2)_nOH \qquad (1)$$

***calculée à partir de l'équation Mth = 18 + 44 x n

Les résultats présentés dans le tableau 2 montrent que les compositions contenant 10% en poids de PEG-90 et celles contenant 10% en poids de PEG-454 sont des compositions conformes à l'invention, car l'application en prétraitement de telles compositions conduisent à un effet antipénétration. Ces compositions sont de plus des compositions préférées selon l'invention car elles conduisent en outre à l'absence d'effet soies.

Les compositions à base de PEG-6, PEG-45 ou PEG-795 ne sont pas conformes à l'invention car l'application de ces compositions en prétraitement ne conduit pas à un effet antipénétration.

EXEMPLE 2

On a réalisé des essais de coloration de soies d'oreilles de porc, sur lesquelles on a appliqué le prétraitement avant coloration, une solution alcoolique contenant 20 % en poids de polyéthylène glycol-90 par rapport au volume total de la composition.

Les résultats de ces essais (évaluation des effets antipénétration et soies) sont rassemblés dans le tableau 3 ci-après.

TABLEAU 3

| Solution contenant 20 % en poids* de polyéthylène glycol** | Masse moléculaire moyenne d'après fournisseur (en $g.mol^{-1}$) | Masse moléculaire théorique*** (en $g.mol^{-1}$) | n** | Effet anti-pénétration | Effet soies |
|---|---|---|---|---|---|
| PEG-90 | 3500-4500 | 4000 | 90 | + | - |

*Les pourcentages sont exprimés en poids par rapport au volume total de la composition.
** nombre de groupements oxydes éthylène dans un polyéthylène glycol répondant à la formule (1) :

$$H(OCH_2CH_2)_n\ OH \qquad (1)$$

***calculée à partir de l'équation Mth = 18 + 44 x n

Les résultats présentés dans le tableau 2 montrent que les compositions comprenant 20 % en poids de PEG-90 conduisent à un effet anti-pénétration. L'application en prétraitement de telles compositions conduit à l'apparition d'un effet soies empêchant la coloration.

EXEMPLE 3

On a réalisé des essais de coloration de soies d'oreilles de porc, sur lesquelles on a appliqué, en prétraitement avant coloration, une solution alcoolique contenant 5 % en poids de PEG-90 par rapport au volume total de la composition. Les résultats de ces essais (évaluation des effets antipénétration et soies) sont rassemblés dans le tableau 4 ci-après.

TABLEAU 4

| Solution contenant 5% en poids* du polyéthylène glycol | Effet anti-pénétration | Effet soies |
|---|---|---|
| PEG-90* | 0 | 0 |
| *Les concentrations sont des pourcentages pondéraux par rapport au volume total de la composition. | | |

Les résultats présentés dans le tableau 3 montrent qu'une composition contenant 5% en poids de PEG 4000 n'est pas conforme à l'invention, car l'application de cette composition en prétraitement ne conduit pas à un effet anti-pénétration.

## Revendications

**1.** Composition anti-pénétrante de prétraitement capillaire comprenant, dans un milieu physiologiquement acceptable, une quantité efficace supérieure à 5 % inférieur à 20 % en poids du poids par rapport au volume total de la composition d'au moins un polymère oxyéthyléné choisi dans le groupe constitué par :

a) les polyéthylène glycol répondant à la formule générale (1) :

$$H(OCH_2CH_2)_nOH \qquad (1)$$

où n est supérieur à 45 et inférieur à 795,

**2.** Composition selon la revendication 1, **caractérisée en ce que** la quantité de polymère oxyéthyléné dans la composition est inférieure à 15% en poids et mieux de l'ordre de 10 % en poids par rapport au volume total de la composition.

**3.** Composition selon la revendication 1 ou 2, **caractérisée en ce que** le polyéthylène glycol a) comprend 75 à 600 groupes oxyde d'éthylène par molécule.

**4.** Composition selon la revendication 3, **caractérisée en ce que** le polyéthylène glycol est choisi parmi les polyéthylène glycol PEG-90, PEG-100, PEG-135, PEG-180, PEG-200, PEG-240, PEG-350 et PEG-454.

**5.** Composition selon l'une quelconque des revendications précédentes, **caractérisée en ce que** le milieu physiologiquement acceptable est un milieu solubilisant du polymère oxyéthyléné, de préférence à propriété bactériologique.

**6.** Composition selon l'une quelconque des revendications précédentes, **caractérisée en ce que** le milieu physiologiquement acceptable comprend un solvant ou un mélange de solvant du polymère oxyéthyléné.

**7.** Composition selon la revendication 6, **caractérisée en ce que** le solvant est choisi parmi l'eau, les alcools, les éthers, le diméthylsulfoxyde, la N-méthylpyrrolidone, les acétones, et leurs mélanges.

**8.** Composition selon la revendication 7, **caractérisée en ce que** les alcools sont choisis parmi les alcanols, l'alcool benzylique ou les alcanediols.

**9.** Composition selon la revendication 8, **caractérisée en ce que** les alcanols sont des alcanols inférieurs ($C_1$-$C_6$), de préférence l'éthanol et l'isopropanol.

**10.** Composition selon la revendication 8, **caractérisée en ce que** les alcanediols sont choisis parmi l'éthylène glycol, le propylène glycol et le pentanediol.

**11.** Composition selon la revendication 7, **caractérisée en ce que** le solvant est un mélange eau/alcool.

**12.** Composition selon la revendication 11, **caractérisée en ce que** l'alcool représente jusqu'à 80 % en volume du mélange eau/alcool.

**13.** Composition selon la revendication 12, **caractérisée en ce que** le mélange eau/alcool est un mélange eau/éthanol comprenant 70% en volume d'éthanol par rapport au poids du mélange eau/éthanol.

**14.** Composition selon l'une quelconque des revendications précédentes, **caractérisée en ce qu'**elle contient en outre des adjuvants cosmétiques choisis parmi les gélifiants et/ou épaississants classiques, les tensioactifs anioniques, non ioniques, cationiques ou amphotères, les agents propénétrants, les émulsionnants, les parfums, les conservateurs, les charges, les filtres solaires, les protéines, les vitamines, les provitamines, les polymères non fixants anioniques, non ioniques, cationiques ou amphotères, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les bactéricides, les séquestrants, les anti-pelliculaires, les agents alcalinisants, les silicones volatiles ou non, linéaires ou cycliques, modifiées ou non, les polyols, et tout autre additif classiquement utilisé dans les compositions cosmétiques destinées à être appliquées sur les cheveux.

**15.** Procédé pour limiter la pénétration dans la peau et/ou les fibres kératiniques d'au moins un colorant, de préférence un colorant d'oxydation, contenu dans une composition de coloration capillaire, **caractérisé en ce qu'**il consiste à appliquer sur le cuir chevelu et sur les cheveux en prétraitement, avant un processus de coloration des cheveux utilisant la composition de coloration capillaire, une composition anti-pénétrante de prétraitement capillaire telle que définie selon l'une quelconque des revendications 1 à 14.

**16.** Procédé selon la revendication 15, **caractérisé en ce que** la durée d'application sur le cuir chevelu et sur les cheveux de la composition antipénétrante est de 5 secondes à une heure, et de préférence de 1 à 8 minutes.

**17.** Procédé selon la revendication 15 ou 16, **caractérisé en ce que** le colorant d'oxydation est choisi parmi les bases d'oxydation, les coupleurs, les orthodiphénols et leurs mélanges.

**18.** Procédé selon la revendication 17, **caractérisé en ce que** les bases d'oxydation sont choisies parmi les ortho- et para-phénylène diamines, les bases doubles, les ortho- et para-aminophénols, les bases hétérocycliques, ainsi que les sels d'addition de tous ces composés avec un acide.

**19.** Procédé selon la revendication 18, **caractérisé en ce que** les bases d'oxydation sont choisies parmi les para-phénylènediamines répondant à la formule (4) :

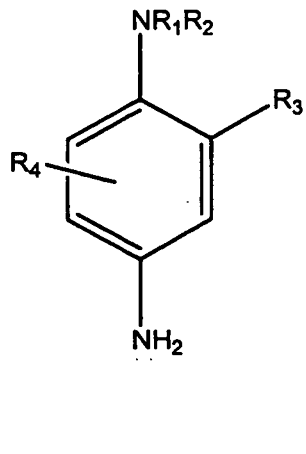

(4)

dans laquelle :

$R_1$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;

$R_2$ représente un atome d'hydrogène, un radical alkyle en $C_1$-$C_4$. monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;

$R_3$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$ ;

$R_4$ représente un atome d'hydrogène, d'halogène ou un radical alkyle en $C_1$-$C_4$ ;

$R_1$ et $R_2$ peuvent également former avec l'atome d'azote qui les porte un hétérocycle azoté à 5 ou 6 chaînons éventuellement substitué par un ou plusieurs groupements alkyle, hydroxy ou uréido.

**20.** Procédé selon la revendication 19, **caractérisé en ce que** les paraphénylène diamines sont choisies parmi la paraphénylènediamine, la paratoluylène diamine, la 2-isopropyl-paraphénylènediamine, la 2-β-hydroxyéthyl-paraphénylènediamine, la 2,6-diméthyl-paraphénylènediamine, la N,N-bis-(β-hydroxyéthyl)-paraphénylènediamine, la 2-chloro-paraphénylènediamine, et leurs sels d'addition avec un acide.

**21.** Procédé selon la revendication 18, **caractérisé en ce que** les bases d'oxydation sont des bases doubles répondant à la formule (5) :

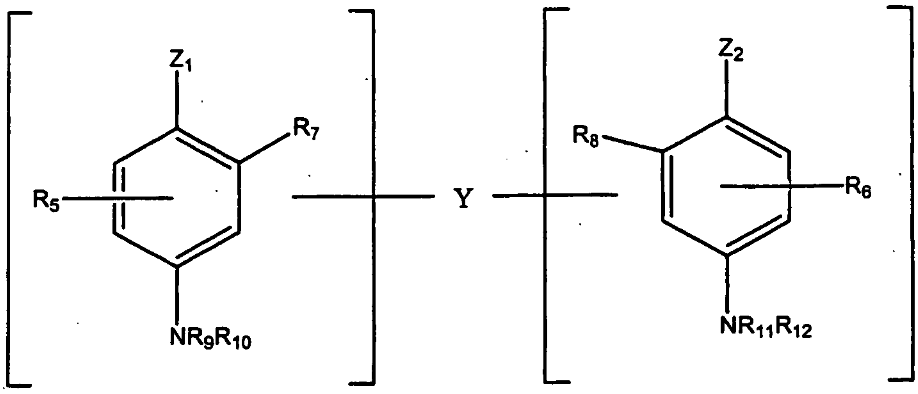

(5)

dans laquelle :

$Z_1$ et $Z_2$, identiques ou différents, représentent un radical hydroxyle ou $-NH_2$ pouvant être substitué par un radical alkyle en $C_1$-$C_4$ ou par un bras de liaison Y;
le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
$R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
$R_7$, $R_8$, $R_9$, $R_{10}$, $R_{11}$ et $R_{12}$, identiques ou différents, représentent un atome d'hydrogène, un bras de liaison Y ou un radical alkyle en $C_1$-$C_4$ ;
étant entendu que les composés de formule (5) ne comportent qu'un seul bras de liaison Y par molécule.

**22.** Procédé selon la revendication 21, **caractérisé en ce que** les bases doubles sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acides.

**23.** Procédé selon la revendication 18, **caractérisé en ce que** les bases d'oxydation sont des para-aminophénols répondant à la formule (6) :

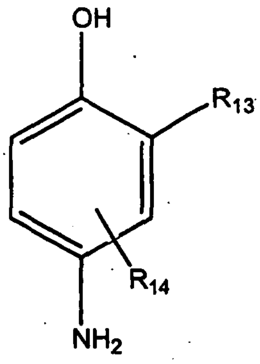

(6)

dans laquelle :

- $R_{13}$ représente un atome d'hydrogène, un atome d'halogène tel que le fluor, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl($C_1$-$C_4$) aminoalkyle en $C_1$-$C_4$ ;
- $R_{14}$ représente un atome d'hydrogène ou un atome d'halogène tel que le fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$)

**24.** Procédé selon la revendication 23, **caractérisé en ce que** les para-aminophénols sont choisis parmi le para-aminophénol, le 4-amino-3-méthylphénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

**25.** Procédé selon la revendications 18, **caractérisé en ce que** les bases d'oxydation sont des orthodiphénols choisis parmi le 2-amino-phénol, le 2-amino-1-hydroxy-5-méthyl-benzène, le 2-amino-1-hydroxy-6-méthylbenzène, le 5-acétamido-2-amino-phénol, et leurs sels d'addition avec un acide.

**26.** Procédé selon la revendication 18, **caractérisé en ce que** les bases d'oxydation sont des bases hétérocycliques choisies parmi les dérivés pyridiniques, les dérivés pyrimidiniques, les dérivés pyrazoliques, et leurs sels d'addition avec un acide.

**27.** Procédé selon la revendication 26, **caractérisé en ce que** les dérivés pyridiniques sont choisis parmi la 2,5-diamino-pyridine, la 2-(4-méthoxyphényl)amino-3-amino-pyridine, la 2,3-diamino-6-méthoxy-pyridine, la 2-(β-méthoxyéthyl) amino-3-amino-6-méthoxy pyridine, et la 3,4-diamino-pyridine.

**28.** Procédé selon la revendication 26, **caractérisé en ce que** les dérivés pyrimidiniques sont choisis parmi la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthylpyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino pyrazolo-[1,5-a]-pyrimidine; et leurs sels d'addition et leurs formes tautomères, lorsqu'il existe un équilibre tautomérique.

**29.** Procédé selon la revendication 26, **caractérisé en ce que** les dérivés pyrazoliques sont choisis parmi le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl pyrazole, le 4,5-diamino-1-tert-butyl-3-méthylpyrazole, le 4,5-diamino-1-(β-hydroxyéthyl)-3-méthyl pyrazole, le 4,5-diamino-1-(β-hydroxyéthyl)- pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, et le 3,5-diamino-4-(β-hydroxyéthyl)amino-1-méthyl-pyrazole.

**30.** Procédé selon l'une quelconque des revendications 18 à 29, **caractérisée en ce que** la base d'oxydation représente de 0,0005 à 12 % en poids, et de préférence de 0,005 à 8 % en poids du poids total de la composition.

**31.** Procédé selon la revendication 17, **caractérisé en ce que** les orthodiphénols comportent un cycle benzénique ou un cycle aromatique condensé portant au moins deux groupes hydroxyle sur deux atomes de carbone consécutifs du cycle.

**32.** Procédé selon la revendication 31, **caractérisé en ce que** les orthodiphénols sont des composés de formule :

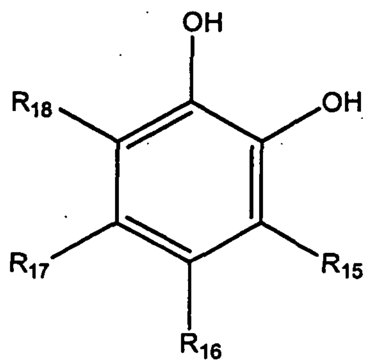

(7)

dans laquelle les substituants $R_{15}$ à $R_{18}$, identiques ou différents, représentent un atome d'hydrogène, un halogène, un radical hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvant être éventuellement substitué, aryle, aryle substitué, un radical hétérocyclique éventuellement substitué, un radical contenant éventuellement un ou plusieurs atomes de silicium, où deux des substituants $R_{15}$ à $R_{18}$ peuvent former conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

**33.** Procédé selon la revendication 31, **caractérisé en ce que** les orthodiphénols sont choisis parmi les flavanols, les flavonols, les anthocyaninidines, les anthocyanines, les hydroxybenzoates, les flavones, les iridoïdes, ces composés pouvant être éventuellement osylés et/ou sous forme d'oligomères, les hydroxystilbènes éventuellement osylés, la 3,4-dihydroxyphénylalanine et ses dérivés, la 2,3-dihydroxyphénylalanine et ses dérivés, la 4,5-dihydroxyphénylalanine et ses dérivés, le 4,5-dihydroxyindole et ses dérivés, le 5,6-dihydroxyindole et ses dérivés, le 6,7-dihydroxyindole et ses dérivés, le 2,3-dihydroxyindole et ses dérivés, les dihydroxycinnamates, les hydroxycoumarines, les hydroxyisocoumarines, les hydroxycoumarones, les hydroxyisocoumarones, les hydroxychalcones, les hydroxychromones, les anthocyanes, les quinones, les hydroxyxantones, et les mélanges de deux ou plus des composés précédents.

**34.** Procédé selon la revendication 33, **caractérisé en ce que** les orthodiphénols sont choisis parmi le 5,6-dihydroxyindole et l'acide 5,6 dihydroxyindole carboxylique.

**35.** Procédé selon l'une des revendications 17 à 34, **caractérisé en ce que** les orthodiphénols sont contenus dans des extraits de plantes, de fruits, ou d'agrumes, ou encore dans des mélanges de ces extraits.

**36.** Procédé selon la revendication 35, **caractérisé en ce que** les orthodiphénols sont contenus dans des extraits de

thé, de raisin, de pomme, de banane, ou de pomme de terre, ou encore dans des mélanges de ces extraits.

**37.** Procédé selon la revendication 17 **caractérisé en ce que** les coupleurs sont choisis parmi les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

**38.** Procédé selon la revendication 37, **caractérisé en ce que** les coupleurs sont choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

**39.** Procédé selon la revendication 37 ou 38, **caractérisé en ce que** le ou les coupleurs représentent de 0,0001 à 15% en poids du poids total de la composition, et de préférence de 0,001 à 10%.

**40.** Procédé selon l'une des revendications 15 à 39, **caractérisé en ce que** la composition de coloration capillaire comprend un ou plusieurs colorants directs, choisis de préférence parmi les colorants nitrés, azoïques ou anthraquinoniques, neutres, cationiques ou anioniques.

**41.** Procédé selon la revendication 40 **caractérisé en ce que** le ou les colorants directs représentent de 0,001 à 20% en poids, de préférence de 0,01 à 10% en poids du poids total de la composition.

**42.** Procédé selon l'une quelconque des revendications 15 à 47,
**caractérisé en ce que** la composition de coloration capillaire comprend un ou plusieurs acides aminés et/ou une ou plusieurs protéines.

**43.** Procédé selon la revendication 42, **caractérisé en ce que** le ou les acides aminés comprennent au moins un groupe thiol et sont choisis parmi les acides aminés ayant une fonction amine en position α par rapport à une fonction acide carboxylique.

**44.** Procédé selon la revendication 43, **caractérisé en ce que** le ou les acides aminés sont choisis parmi la cystéine et ses dérivés, les protéines sont choisies parmi le glutathion et ses dérivés.

**45.** Procédé selon l'une des revendications 40 à 44, **caractérisé en ce que** le rapport molaire du (ou des) acide(s) aminé(s) et du (ou des) protéines au(x) colorant(s) d'oxydation varie de 0,001 à 50, de préférence de 0,01 à 5, et mieux de 0,05 à 2,5.

**46.** Procédé selon l'une quelconque des revendications 15 à 45, **caractérisé en ce que** la composition de coloration capillaire comprend en outre une enzyme.

**47.** Procédé selon la revendication 46, **caractérisé en ce que** l'enzyme est choisie parmi les pyranose oxydases, les glucose oxydases, les glycérol oxydases, les lactate oxydases, les pyruvate oxydases, les uricases, les choline oxydases, les sarcosine oxydases, les bilirubine oxydases, les laccases, les tyrosinases, les péroxydases, les catalases, les superoxyde dimutases et leurs mélanges, ou parmi des extraits végétaux ou animaux contenant des enzymes précitées.

**48.** Procédé selon la revendication 47, **caractérisé en ce que** l'enzyme est choisie parmi les tyrosinases.

**49.** Procédé selon la revendication 47 ou 48, **caractérisé en ce que** la composition de coloration capillaire comprend $5.10^{-3}$ à 5 mg, de préférence $5.10^{-2}$ à 0,5 mg d'enzyme par millilitre de composition finale.

**50.** Procédé selon l'une quelconque des revendications 15 à 49, **caractérisé en ce que** le colorant d'oxydation est présent en une quantité de 1 mM à 10 mM par litre de composition.

**51.** Procédé selon l'une quelconque des revendications 15 à 50,
**caractérisé en ce que** la composition de coloration capillaire comporte également une quantité effective d'un système comprenant un premier constituant choisi parmi les sels et oxydes de Mn(II) et/ou de Zn(II) et leurs mélanges et un second constituant choisi parmi les hydrogénocarbonates alcalins, les hydrogénocarbonates alcalino-terreux et leurs mélanges, les proportions du premier et du second constituant étant telles que :

$$\frac{[Mn(II)]}{[HCO_3]} \leq 1 \text{ avec } [Mn(II)] \neq 0$$

$$\frac{[Zn(II)]}{[HCO_3]} \leq 1 \text{ avec } [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

où [Mn(II)], [Zn(II)] et $[HCO_3]$ représentent respectivement les concentrations molaires en Mn(II), Zn(II) et $HCO_3$ dans la composition

**52.** Procédé selon la revendication 51, **caractérisé en ce que** le rapport $\frac{[Mn(II)]}{[HCO_3]}$ varie de $10^{-5}$ à $10^{-1}$, de préférence de $10^{-3}$ à $10^{-2}$, et mieux est de l'ordre de $5.10^{-3}$.

**53.** Procédé selon la revendication 51 ou 52 **caractérisé en ce que** le rapport $\frac{[Zn(II)]}{[HCO_3]}$ varie de $10^{-4}$ à 1, de préférence de $10^{-3}$ à 1, et mieux est de l'ordre de $5.10^{-1}$.

**54.** Procédé selon l'une quelconque des revendications 51 à 53,
**caractérisé en ce que** le rapport $\frac{[Mn(II) + Zn(II)]}{[HCO_3]}$ varie de $10^{-5}$ à $10^{-1}$, de préférence $10^{-3}$ à $10^{-2}$.

**55.** Procédé selon l'une quelconque des revendications 51 à 53, **caractérisée en ce que** les sels de Mn(II) et de Zn (II) sont choisis parmi les chlorure, fluorure, iodure, sulfate, phosphate, nitrate, perchlorate, les sels d'acides carboxyliques et leurs mélanges.

**56.** Procédé selon l'une quelconque des revendications 51 à 55,
**caractérisé en ce que** le sel de Mn(II) et/ou de Zn(II) est le chlorure.

**57.** Procédé selon l'une quelconque des revendications 51 à 56,
**caractérisé en ce que** les sels d'acides carboxyliques sont des sels d'acides carboxyliques hydroxylés.

**58.** Procédé selon l'une quelconque des revendications 51 à 56,
**caractérisé en ce que** le sel d'acide carboxylique hydroxylé est le gluconate.

**59.** Procédé selon l'une quelconque des revendications 51 à 58,
**caractérisée en ce que** l'hydrogénocarbonate est choisi parmi l'hydrogénocarbonate de sodium, l'hydrogénocarbonate de potassium et leurs mélanges.

## Claims

**1.** A hair pre-treatment anti-penetration composition comprising, in a physiologically acceptable medium, an efficient amount higher than 5 % and lower than 20% in weight of the weight based on the total volume of the composition of at least one oxyethylenated polymer selected in the group constituted of:

a) polyethylene glycols with the general formula (1):

$$H(OCH_2CH_2)_nOH \qquad (1)$$

where n is higher than 45 and lower than 795.

**2.** A composition according to claim 1, **characterized in that** the oxyethylenated polymer amount in the composition is lower than 15% in weight and more preferably in the order of 10 % in weight based on the total volume of the composition.

**3.** A composition according to claim 1 or 2. **characterized in that** the polyethylene glycol a) comprises 75 to 600 ethylene oxide groups per molecule.

**4.** A composition according to claim 3, **characterized in that** the polyethylene glycol is selected amongst the polyethylene glycols PEG-90, PEG-100, PEG-135, PEG-180, PEG-200, PEG-240, PEG-350 and PEG-454.

**5.** A composition according to any of the preceding claims, **characterized in that** the physiologically acceptable medium is a solubilizing medium for the oxyethylenated polymer, preferably having a bacteriological property.

**6.** A composition according to any of the preceding claims, **characterized in that** the physiologically acceptable medium comprises a solvent or a mixture of solvents for the oxyethylenated polymer.

**7.** A composition according to claim 6, **characterized in that** the solvent is selected amongst water, alcohols, ethers, dimethylsulfoxide, N-methylpyrrolidone, acetones, and the mixtures thereof.

**8.** A composition according to claim 7, **characterized in that** the alcohols are selected amongst alkanols, benzyl alcohol or alkanediols.

**9.** A composition according to claim 8, **characterized in that** the alkanols are ($C_1$-$C_6$) lower alkanols, preferably ethanol and isopropanol.

**10.** A composition according to claim 8, **characterized in that** the alkanediols are selected amongst ethylene glycol, propylene glycol and pentanediol.

**11.** A composition according to claim 7, **characterized in that** the solvent is a water/alcohol mixture.

**12.** A composition according to claim 11, **characterized in that** the alcohol accounts up to 80 % in volume of the water/alcohol mixture.

**13.** A composition according to claim 12, **characterized in that** the water/alcohol mixture is a water/ethanol mixture comprising 70% in volume of ethanol based on the weight of the water/ethanol mixture.

**14.** A composition according to any of the preceding claims, **characterized in that** it additionally contains cosmetic builders selected amongst conventional gellants and/or thickening agents, anionic, non ionic, cationic or amphoteric surfactants, propenetrating agents, emulsifiers, perfumes, preservatives, fillers, sunscreens, proteins, vitamins, provitamins, anionic, non ionic, cationic or amphoteric non fixing polymers, hydrating agents, emollients, softening agents, mineral, vegetable or synthetic oils, hydrophilic or lipophilic active ingredients such as ceramides and pseudoceramides, anti-foaming agents, antiperspirant agents, anti-free radical agents, bactericides, sequestrants, anti-dandruff agents, alkalinizing agents, volatile or non volatile, linear or cyclic, modified or not, silicones, polyols, and any other additive conventionally used in cosmetic compositions intended

to be applied on the hair.

**15.** A method for limiting the penetration into the skin and/or the keratin fibres of at least one dye, preferably one oxydation dye, contained in a hair dyeing composition, **characterised in that** comprises applying on the hair scalp and on the hair, as a pre-treatment, before a hair dyeing process using the hair dyeing composition, a hair pre-treatment anti-penetration compositions such as defined according to any of claims 1 to 14.

**16.** A method according to claim 15, **characterized in that** the application duration on the hair scalp and on the hair of the antipenetrating composition is from 5 seconds to one hour, and preferably from 1 to 10 minutes.

**17.** A method according to claim 15 or 16, **characterized in that** oxidation dye is selected amongst oxidation bases, coupling agents, orthodiphenols and the mixtures thereof.

**18.** A method according to claim 17, **characterized in that** the oxidation bases are selected amongst ortho- and para-phenylene diamines, double bases, ortho- and para-aminophenols, heterocyclic bases, as well as the addition salts of all those compounds with an acid.

**19.** A method according to claim 18, **characterized in that** the oxidation bases are selected amongst para-phenylenediamines having the formula (4):

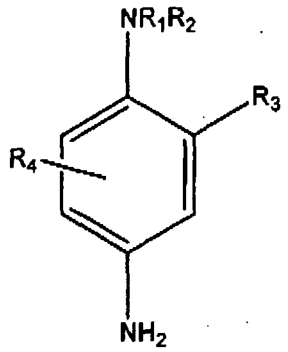

(4)

where:

$R_1$ represents a hydrogen atom, a $C_1$-$C_4$ alkyl moiety, a $C_1$-$C_4$ monohydroxyalkyle, $C_2$-$C_4$ polyhydroxyalkyl, ($C_1$-$C_4$)alkoxy($C_1$-$C_4$)alkyl, $C_1$-$C_4$ alkyl substituted by a nitrogen, phenyl or 4'-aminophenyl group;
$R_2$ represents a hydrogen atom, $C_1$-$C_4$ alkyl radical, a $C_1$-$C_4$ monohydroxyalkyl or $C_2$-$C_4$ polyhydroxyalkyle, ($C_1$-$C_4$)alkoxy($C_1$-$C_4$)alkyl or $C_1$-$C_4$ alkyl substituted by a nitrogen group;
$R_3$ represents a hydrogen atom, a halogen atom such as a chlorine atom, a $C_1$-$C_4$ alkyl moiety, a sulfo, carboxy, a $C_1$-$C_4$ monohydroxyalkyle or $C_1$-$C_4$ hydroxyalkoxy, $C_1$-$C_4$ acetylaminoalkoxy, $C_1$-$C_4$ mesylaminoalkoxy or $C_1$-$C_4$ carbamoylaminoalkoxy;
$R_4$ represents a hydrogen, halogen atom or a $C_1$-$C_4$ alkyl moiety;
$R_1$ and $R_2$ may also form with the nitrogen atom carrying them a 5 or 6 members nitrogen heteroring optionally substituted by one or more alkyl, hydroxy or ureido groups.

**20.** A method according to claim 19, **characterized in that** the para-phenylenediamines are selected amongst paraphenylenediamine, paratoluylenediamine, 2-isopropyl-paraphenylenediamine, 2-β-hydroxyethylparaphenylenediamine, 2,6-dimethyl-paraphenylenediamine,N,N-bis-(β-hydroxyethyl)-paraphenylenediamine, 2-chloro-paraphenylenediamine, and their addition salts with an acid.

**21.** A method according to claim 18, **characterized in that** the oxidation bases are double bases having the formula (5):

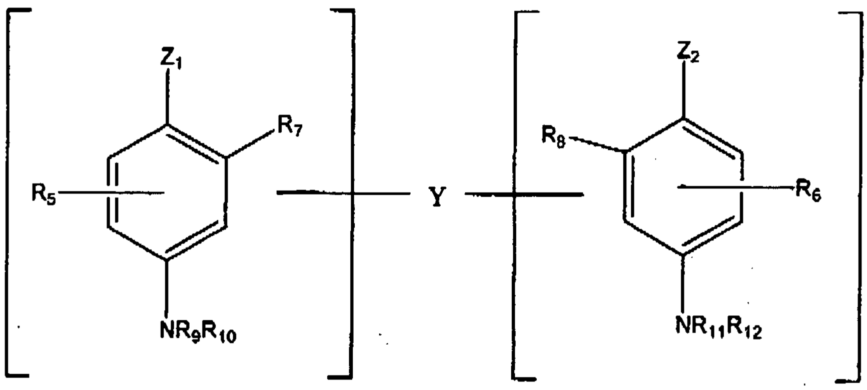

(5)

where:

- $Z_1$ and $Z_2$, whether identical or different, represent a hydroxyl or $-NH_2$ moiety optionally substituted by a $C_1$-$C_4$ alkyl moiety or by a Y binding branch;
- the Y binding branch represents an alkylene chain comprising 1 to 14 carbon atoms, linear or branched, able to be interrupted or ending with one or more nitrogen groups and/or by one or more heteroatoms such as oxygen, sulphur or nitrogen atoms, and optionally substituted by one ore more $C_1$-$C_6$ hydroxyl or alkoxy moieties;
- $R_5$ and $R_6$ represent a hydrogen or a halogen atom, a $C_1$-$C_4$ alkyl, $C_1$-$C_4$ monohydroxyalkyl, $C_2$-$C_4$ polyhydroxyalkyl, $C_1$-$C_4$ aminoalkyl moiety or a Y binding branch;
- $R_7$, $R_8$, $R_9$, $R_{10}$, $R_{11}$ and $R_{12}$, whether identical or different, represent a hydrogen atom, a Y binding branch or a $C_1$-$C_4$ alkyl moiety; on the condition that the compounds with formula (5) only have one Y binding branch per molecule.

**22.** A method according to claim 21, **characterized in that** the double bases are selected amongst N,N'-bis-(β-hydroxyethyl) N,N'-bis-(4'-aminophenyl) 1,3-diamino propanol, N,N'-bis-(β-hydroxyethyl) N,N'-bis-(4'-aminophenyl) ethylenediamine, N,N'-bis-(4-aminophenyl) tetramethylenediamine, N,N'-bis-(β-hydroxyethyl) N,N'-bis-(4-aminophenyl) tetramethylenediamine, N,N'-bis-(4-methyl-aminophenyl) tetramethylenediamine, N,N'-bis-(ethyl) N,N'-bis-(4'-amino, 3'-methyl) ethylenediamine, 1,8-bis-(2,5-diaminophenoxy)-3,5-dioxaoctane, and their addition salts with an acid.

**23.** A method according to claim 18, **characterized in that** the oxidation bases are para-aminophenols having the formula (6):

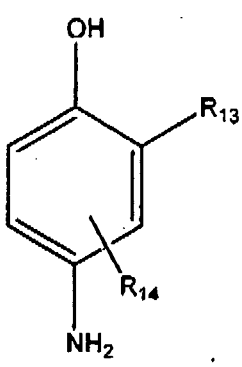

(6)

where:

- $R_{13}$ represents a hydrogen atom, a halogen atom such as fluorine, a $C_1$-$C_4$ alkyl, $C_1$-$C_4$ monohydroxyalkyl, $(C_1$-$C_4)$ alcoxy$(C_1$-$C_4)$ alkyl, or $C_1$-$C_4$ aminoalkyl, or $C_1$-$C_4$ hydroxyalkyl aminoalkyl $(C_1$-$C_4)$ moiety; and

• $R_{14}$ represents a hydrogen atom or a halogen atom such as fluorine, a $C_1$-$C_4$ alkyl, $C_1$-$C_4$ monohydroxyalkyle, $C_2$-$C_4$ polyhydroxyalkyle, $C_1$-$C_4$ aminoalkyl, $C_1$-$C_4$ cyanoalkyl or ($C_1$-$C_4$)alkoxy($C_1$-$C_4$)alkyl moiety.

**24.** A method according to claim 23, **characterized in that** the para-aminophenols are selected amongst para-aminophenol, 4-amino-3-methyl-phenol, 4-amino-3-fluoro-phenol, 4-amino-3-hydroxymethyl-phenol, 4-amino-2-methylphenol, 4-amino-2-hydroxymethyl-phenol, 4-amino-2-methoxymethyl-phenol, 4-amino-2-aminomethyl-phenol, 4-amino-2-(β-hydroxyethyl-aminomethyl)-phenol, and their addition salts with an acid.

**25.** A method according to claim 18, **characterized in that** the oxidation bases are orthodiphenols selected amongst 2-amino-phenol, 2-amino-1-hydroxy-5-methyl-benzene, 2-amino-1-hydroxy-6-methyl-benzene, 5-acetamido-2-amino-phenol, and their addition salts thereof with an acid.

**26.** A method according to claim 18, **characterized in that** the oxidation bases are heterocyclic bases selected amongst pyridine derivates, pyrimidine derivates, pyrazoline derivates, and their addition salts with an acid.

**27.** A method according to claim 26, **characterized in that** the pyridine derivates are selected amongst 2,5-diamino-pyridine, 2-(4-methoxyphenyl)amino-3-amino-pyridine, 2,3-diamino-6-methoxy-pyridine, 2-(β-methoxyethyl)amino-3-amino-6-methoxy pyridine, and 3,4-diamino-pyridine.

**28.** A method according to claim 26, **characterized in that** the pyrimidine derivates are selected amongst 2,4,5,6-tetra-aminopyrimidine, 4-hydroxy-2,5,6-triaminopyrimidine, 2-hydroxy-4,5,6-triaminopyrimidine, 2,4-dihydroxy-5,6-diaminopyrimidine, 2,5,6-triaminopyrimidine, and the pyrazolo-pyrimidine derivates such as those mentioned in Patent Application FR-A-2 750 048 and amongst which one can mention pyrazolo-[1,5-a]-pyrimidine-3,7-diamine; 2,5-dimethylpyrazolo-[1,5-a]-pyrimidine-3,7-diamine; pyrazolo-[1,5-a]-pyrimidine-3,5-diamine; 2,7-dimethyl-pyrazolo-[1,5-a]-pyrimidine-3,5-diamine; 3-amino-pyrazolo-[1,5-a]-pyrimidin-7-ol; 3-amino-pyrazolo-[1,5-a]-pyrimidin-5-ol; 2-(3-amino pyrazolo-[1,5-a]-pyrimidin-7-ylamino)-ethanol; 2-(7-amino-pyrazolo-[1,5-a]-pyrimidin-3-ylamino)-ethanol; 2-[(3-amino-pyrazolo[1,5-a]pyrimidin-7-yl)-(2-hydroxy-ethyl)-amino]-ethanol; 2-[(7-amino-pyrazolo-[1,5-a]pyrimidin-3-yl)-(2-hydroxy-ethyl)-amino]-ethanol; 5,6-dimethyl-pyrazolo-[1,5-a]-pyrimidine-3,7-diamine; 2,6-dimethyl-pyrazolo-[1,5-a]-pyrimidine-3,7-diamine; 2,5,N7,N7-tetramethyl-pyrazolo-[1,5-a]-pyrimidine-3,7-diamine; 3-amino-5-methyl-7-imidazolylpropylamino pyrazolo-[1,5-a]-pyrimidine; and the addition salts thereof and their tautomeric forms, when there is a tautomeric balance.

**29.** A method according to claim 26, **characterized in that** the pyrazole derivates are selected amongst 4,5-diamino-1-methyl-pyrazole, 3,4-diamino-pyrazole, 4,5-diamino-1-(4'-chlorobenzyl)-pyrazole, 4,5-diamino 1,3-dimethylpyrazole, 4,5-diamino-3-methyl-1-phenyl pyrazole, 4,5-diamino 1-methyl-3-phenylpyrazole, 4-amino-1,3-dimethyl-5-hydrazino-pyrazole, 1-benzyl-4,5-diamino-3-methyl-pyrazole, 4,5-diamino-3-tert-butyl-1-methyl pyrazole, 4,5-diamino-1-tert-butyl-3-methyl-pyrazole, 4,5-diamino-1-(β-hydroxyethyl)-3-methyl pyrazole, 4,5-diamino-1-(β-hydroxyethyl)- pyrazole, 4,5-diamino-1-ethyl-3-methyl-pyrazole, 4,5-diamino-1-ethyl-3-(4'-methoxyphenyl)-pyrazole, 4,5-diamino-1-ethyl-3-hydroxymethyl-pyrazole, 4,5-diamino-3-hydroxymethyl-1-methyl-pyrazole, 4,5-diamino-3-hydroxymethyl-1-isopropyl-pyrazole, 4,5-diamino-3-methyl-1-isopropylpyrazole, 4-amino-5-(2'-aminoethyl)amino-1,3-dimethyl-pyrazole, ,4,5-triamino-pyrazole, 1-methyl-3,4,5-triamino-pyrazole, 3,5-diamino-1-methyl-4-methylaminopyrazole, and 3,5-diamino-4-(β-hydroxyethyl)amino-1-methyl-pyrazoie.

**30.** A method according to any of claims 18 to 29, **characterized in that** the oxidation base accounts for 0.0005 to 12 % in weight, more preferably from 0.005 to 8 % in weight of the total weight of the composition.

**31.** A method according to claim 17, **characterized in that** the orthodiphenols comprise a condensed benzene ring or an aromatic ring carrying at least two hydroxyl groups on two consecutive carbon atoms of the ring.

**32.** A method according to claim 31, **characterized in that** the orthodiphenols are compounds having the formula:

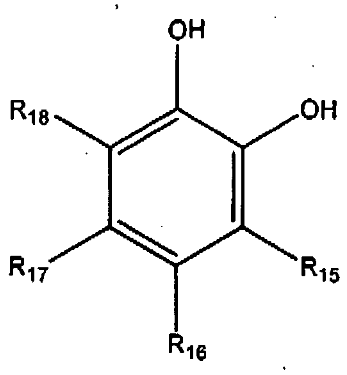

(7)

where the $R_{15}$ to $R_{18}$ substituents, identical or different, represent a hydrogen atom, a halogen, hydroxyl, carboxyl, alkyl carboxylate, optionally substituted amino moiety, optionally substituted linear or branched alkyl, optionally substituted linear or branched alkenyl, optionally substituted cycloalkyl, alkoxy, alkoxyalkyl, alkoxyaryl, the aryl group being optionally substituted, aryl, aryl substituted, an optionally substituted heterocyclic moiety, a moiety optionally containing one or more silicon atoms, where two of the $R_{15}$ to $R_{18}$ substituents form together a saturated or an unsaturated ring optionally containing one or more heteroatoms and optinally condensed with one or more saturated or unsaturated rings optionally containing one or more heteroatoms.

**33.** A method according to claim 31, **characterized in that** the orthodiphenols are selected amongst flavanols, flavonols, anthocyaninidins, anthocyanines, hydroxybenzoates, flavones, iridoids, such compounds being optionally osylated and/or in the form of oligomers, optionally osylated hydroxystilbenes, 3,4-dihydroxyphenylalanine and the derivates thereof, 2,3-dihydroxyphenylalanine and the derivates thereof, 4,5-dihydroxyphenylalanine and the derivates thereof, 4,5-dihydroxyindole and the derivates thereof, 5,6-dihydroxyindole and the derivates thereof, 6,7-dihydroxyindole and the derivates thereof, 2,3-dihydroxyindole and the derivates thereof, dihydroxycinnamates, hydroxycoumarins, hydroxyisocoumarins, hydroxycoumarones, hydroxyisocoumarones, hydroxychalcones, hydroxychromones, anthocyanes, quinones, hydroxyxantones, and the mixtures of two or more of the previous compounds.

**34.** A method according to claim 33, **characterized in that** the orthodiphenols are selected amongst 5,6-dihydroxyindole and 5,6 dihydroxyindole carboxylic acid.

**35.** A method according to one of claims 17 to 34, **characterized in that** the orthodiphenols are contained in plant, fruit or citrus fruit extracts as well as in mixtures of such extracts.

**36.** A method according to claim 35, **characterized in that** the orthodiphenols are contained in tea, grape, apple, banana, or potato extracts, as well as in mixtures of such extracts.

**37.** A method according to claim 17 **characterized in that** the coupling agents are selected amongst meta-aminophenols, meta-phenylenediamines, metadiphenols, naphthols and heterocyclic coupling agents such as for example, indole derivates, indolin derivates, sesamol and the derivates thereof, pyridine derivates, pyrazolotriazole derivates, pyrazolones, indazoles, benzimidazoles, benzothiazoles, benzoxazoles, 1,3-benzodioxoles, quinolins and their addition salts with an acid.

**38.** A method according to claim 37, **characterized in that** the coupling agents are selected amongst 2,4-diamino 1-(β-hydroxyethyloxy) benzene, 2-methyl 5-amino phenol, 5-N-(β-hydroxyethyl)amino 2-methyl phenol, 3-amino phenol, 1,3-dihydroxy benzene, 1,3-dihydroxy 2-methyl benzene, 4-chloro 1,3-dihydroxy benzene, 2-amino 4-(β-hydroxyethylamino) 1-methoxy benzene, 1,3-diamino benzene, 1,3-bis-(2,4-diaminophenoxy) propane, sesamol, 1-amino 2-methoxy 4,5-methylenedioxy benzene, α-naphthol, 6-hydroxy indole, 4-hydroxy indole, 4-hydroxy N-methyl indole, 6-hydroxy indoline, 2,6-dihydroxy 4-methyl pyridine, 1-H 3-methyl pyrazole 5-one, 1-phenyl 3-methyl pyrazole 5-one, 2-amino 3-hydroxypyridine, 3,6-dimethyl-pyrazolo-[3,2-c]-1,2,4-triazole, 2,6-dimethylpyrazolo-[1,5-b]-1,2,4-triazole and the addition salts thereof with an acid.

**39.** A method according to claim 37 or 38, **characterized in that** coupling agent(s) account(s) for 0.0001 to 15% in weight of the total weight of the composition, and preferably from 0.001 to 10%.

**40.** A method according to one of claims 15 to 39, **characterized in that** the hair dyeing composition comprise one or more direct dyes, preferably selected from the nitrated, azoic or anthraquinonic, neutral, cationic or anionic dyes.

**41.** A method according to claim 40 **characterized in that** direct dyes account for 0.001 to 20% in weight, preferably from 0.01 to 10% in weight of the total weight of the composition.

**42.** A method according to any of claims 15 to 41, **characterized in that** the hair dyeing composition comprises one or more amino acids and/or one or more proteins.

**43.** A method according to claim 42, **characterized in that** the amino acids comprise at least one thiol group and are selected amongst amino acids having an amine function in position $\alpha$ compared with a carboxylic acid function.

**44.** A method according to claim 43, **characterized in that** amino acid(s) is/are selected amongst cystein and the derivates thereof, the proteins are selected amongst glutathione and the derivates thereof.

**45.** A method according to one of claims 40 to 44, **characterized in that** the molar ratio of the amino acid(s) and of the protein(s) to the other oxidation dyes varies from 0.001 to 50, preferably from 0.01 to 5, and more preferably from 0.05 to 2.5.

**46.** A method according to any of claims 15 to 45, **characterized in that** the hair dyeing composition further comprises an enzyme.

**47.** A method according to claim 46, **characterized in that** the enzyme is selected amongst pyranose oxydases glucose oxydases, glycerol oxydases, lactate oxidases, pyruvate oxidases, uricases, cholin oxidases, sarcosin oxidases, bilirubin oxydases, laccases, tyrosinases, peroxidases, catalases, superoxyde dimutases and the mixtures thereof, or amongst plant or animal extracts containing the above-mentioned enzymes.

**48.** A method according to claim 47, **characterized in that** the enzyme is selected amongst the tyrosinases.

**49.** A method according to claim 47 or 48, **characterized in that** the hair dyeing composition comprises $5.10^{-3}$ to 5 mg, preferably $5.10^{-2}$ to 0,5 mg of enzyme per millilitre of final composition.

**50.** A method according to any of claims 15 to 49, **characterized in that** the oxidation dye is present in an amount ranging from 1 mM to 10 mM per litre of composition.

**51.** A method according to any claims 15 to 50, **characterized in that** the hair dyeing composition further comprises an effective amount of a system comprising a first component selected amongst the Mn(II) and/or Zn(II) salts and oxide and the mixtures thereof and a second component selected amongst alkaline hydrogenocarbonates, earth alkaline hydrogenocarbonates and the mixtures thereof, the proportions of the first and second component are such that:

$$\frac{[Mn(II)]}{[HCO_3]} \leq 1 \text{ with } [\mathrm{Mn(II)}] \neq 0$$

$$\frac{[Zn(II)]}{[HCO_3]} \leq 1 \text{ with } [\mathrm{Zn(II)}] \neq 0$$

$$\frac{[Mn(II)+Zn(II)]}{[HCO_3]} \leq 1 \text{ with } [\mathrm{Mn(II)}] \text{ and } [\mathrm{Zn(II)}] \neq 0$$

where [Mn(II)], [Zn(II)] and [$HCO_3$] represent respectively the Mn(II), Zn(II) molar concentrations and $HCO_3$ in the composition.

**52.** A method according to claim 51, **characterized in that** the ratio $\frac{[Mn(II)]}{[HCO_3]}$ varies from $10^{-5}$ to $10^{-1}$ preferably from $10^{-3}$ to $10^{-2}$, and more preferably is in the order of $5.10^{-3}$.

**53.** A method according to claim 51 or 52, **characterized in that** the ratio $\frac{[Zn(II)]}{[HCO_3]}$ varies from $10^{-4}$ to 1, preferably from $10^{-3}$ to 1, and more preferably is in the order of $5.10^{-1}$.

**54.** A method according to any of claims 51 to 53, **characterized in that** the ratio $\frac{[Mn(II)+Zn(II)]}{[HCO_3]}$ varies from $10^{-5}$ to $10^{-1}$, preferably $10^{-3}$ to $10^{-2}$.

**55.** A method according to any of claims 51 to 53, **characterized in that** the Mn(II) and Zn(II) salts are selected amongst chloride, fluoride, iodure, sulphate, phosphate, nitrate, perchlorate, and carboxylic acid salts and the mixtures thereof.

**56.** A method according to any of claims 51 to 55, **characterized in that** the Mn(II) and/or Zn(II) salt is a chloride.

**57.** A method according to any of claims 51 to 56, **characterized in that** the carboxylic acid salts are hydroxylated carboxylic acid salts.

**58.** A method according to any of claims 51 to 56, **characterized in that** the hydroxylated carboxylic acid salt is gluconate.

**59.** A method according to any of claims 51 to 58, **characterized in that** the hydrogenocarbonate is selected amongst sodium hydrogenocarbonate, potassium hydrogenocarbonate and the mixtures thereof.

## Patentansprüche

**1.** Antipenetrations-Haarvorbehandlungszusammensetzung, die in einem physiologisch verträglichen Medium eine wirksame Menge von über 5 Gew.-% oder unter 20 Gew.-%, bezogen auf das gesamte Volumen der Zusammensetzung, wenigstens eines oxyethylenierten Polymers, ausgewählt aus der Gruppe, bestehend aus:

a) den Polyethylenglykolen, die der allgemeinen Formel (1) entsprechen:

$$H(OCH_2CH_2)_nOH \qquad (1)$$

worin n über 45 und unter 795 ist,
umfasst.

**2.** Zusammensetzung nach Anspruch 1, **dadurch gekennzeichnet, dass** die Menge an oxyethyleniertern Polymer in der Zusammensetzung unter 15 Gew.-% und besser in der Größenordnung von 10 Gew.-%, bezogen auf das gesamte Volumen der Zusammensetzung, ist.

**3.** Zusammensetzung nach Anspruch 1 oder 2, **dadurch gekennzeichnet, dass** das Polyethylenglykol a) 75 bis 600 Ethylenoxid-Gruppen pro Molekül umfasst.

**4.** Zusammensetzung nach Anspruch 3, **dadurch gekennzeichnet, dass** das Polyethylenglykol unter den Polyethylenglykolen PEG-90, PEG-100, PEG-135, PEG-180, PEG-200, PEG-240, PEG-350 und PEG-454 ausgewählt ist.

**5.** Zusammensetzung nach einem der vorangehenden Ansprüche, **dadurch gekennzeichnet, dass** das physiologisch verträgliche Medium ein Medium ist, das oxyethyleniertes Polymer solubilisiert, vorzugsweise bakteriologische Eigenschaften hat.

**6.** Zusammensetzung nach einem der vorangehenden Ansprüche, **dadurch gekennzeichnet, dass** das physiologisch verträgliche Medium ein Lösungsmittel oder ein Lösungsmittelgemisch für das oxyethylenierte Polymer umfasst.

**7.** Zusammensetzung nach Anspruch 6, **dadurch gekennzeichnet, dass** das Lösungsmittel unter Wasser, Alkoholen, Ethern, Dimethylsulfoxid, N-Methylpyrrolidon, Acetonen und deren Gemischen ausgewählt ist.

**8.** Zusammensetzung nach Anspruch 7, **dadurch gekennzeichnet, dass** die Alkohole unter Alkanolen, Benzylalkohol und Alkandiolen ausgewählt sind.

**9.** Zusammensetzung nach Anspruch 8, **dadurch gekennzeichnet, dass** die Alkanole niedere ($C_1$-$C_6$)-Alkanole, vorzugsweise Ethanol und Isopropanol, sind.

**10.** Zusammensetzung nach Anspruch 8, **dadurch gekennzeichnet, dass** die Alkandiole unter Ethylenglykol, Propylenglykol und Pentandiol ausgewählt sind.

**11.** Zusammensetzung nach Anspruch 7, **dadurch gekennzeichnet, dass** das Lösungsmittel ein Wasser/Alkohol-Gemisch ist.

**12.** Zusammensetzung nach Anspruch 11, **dadurch gekennzeichnet, dass** der Alkohol bis zu 80 Vol.-% des Wasser/Alkohol-Gemisches darstellt.

**13.** Zusammensetzung nach Anspruch 12, **dadurch gekennzeichnet, dass** das Wasser/Alkohol-Gemisch ein Wasser/Ethanol-Gemisch ist, das 70 Vol.-% Ethanol, bezogen auf das Gewicht des Wasser/Ethanol-Gemisches, umfasst.

**14.** Zusammensetzung nach einem der vorangehenden Ansprüche, **dadurch gekennzeichnet, dass** sie außerdem kosmetische Adjuvantien, ausgewählt unter den klassischen Geliermitteln und/oder Verdickungsmitteln, den anionischen, nicht-ionischen, kationischen oder amphoteren oberflächenaktiven Mitteln, den Penetrationsförderungsmitteln, den Emulgatoren, den Parfums, den Konservierungsmitteln, den Füllstoffen, den Sonnenfiltem, den Proteinen, den Vitaminen, den Provitaminen, den nicht-fixierenden anionischen, nicht-ionischen, kationischen oder amphoteren Polymeren, den Hydratisierungsmitteln, den Emollentien, den weichmachenden Mitteln, den mineralischen, pflanzlichen oder synthetischen Ölen, den hydrophilen oder lipophilen Wirkstoffen, wie den Ceramiden und Pseudoceramiden, den Entschäumungs-Mitteln, den Antiperspirantien, den Mitteln gegen freie Radikale, den Bakteriziden, den Sequestriermitteln, den Peeling-Mitteln, den alkalisierenden Mitteln, den flüchtigen oder nicht-flüchtigen Silikonen, die linear oder cyclisch sind, modifiziert sind oder nicht-modifiziert sind, den Polyolen und jedem anderen Additiv, das klassischerweise in den kosmetischen Zusammensetzungen verwendet wird, die dazu bestimmt sind, auf die Haare aufgetragen zu werden, enthält.

**15.** Verfahren zur Begrenzung der Penetration wenigstens einer Farbe, vorzugsweise einer Oxidationsfarbe, die in einer Haarfärbezusammensetzung enthalten ist, in die Haut und/oder die Keratinfasern, **dadurch gekennzeichnet, dass** es darin besteht, auf die behaarte Kopfhaut und die Haare als Vorbehandlung vor einem Färbeprozess der Haare unter Verwendung der Haarfärbezusammensetzung eine Antipenetrations-Haarvorbehandtungszusammensetzung wie sie in einem der Ansprüche 1 bis 14 definiert ist, aufzutragen bzw. anzuwenden.

**16.** Verfahren nach Anspruch 15, **dadurch gekennzeichnet, dass** die Anwendungsdauer auf die behaarte Kopfhaut und auf die Haare für die Antipenetrationszusammensetzung 5 Sekunden bis eine Stunde und vorzugsweise 1 bis 8 Minuten ist.

**17.** Verfahren nach Anspruch 15 oder 16, **dadurch gekennzeichnet, dass** die Oxidationsfarbe unter den Oxidationsbasen, den Kupplungsmitteln, den ortho-Diphenolen und ihren Gemischen ausgewählt ist.

**18.** Verfahren nach Anspruch 17, **dadurch gekennzeichnet, dass** die Oxidationsbasen unter den ortho- und para-Phenylendiaminen, den doppelten Basen, den ortho- und para-Aminophenolen, den heterocyclischen Basen sowie den Additionssalzen aller dieser Verbindungen mit einer Säure ausgewählt sind.

**19.** Verfahren nach Anspruch 18, **dadurch gekennzeichnet, dass** die Oxidationsbasen unter den para-Phenylendiaminen ausgewählt sind, die der Formel (4) entsprechen:

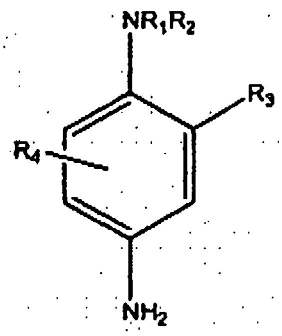

(4)

worin:

$R_1$ ein Wasserstoffatom, einen $C_1$-$C_4$-Alkylrest, einen $C_1$-$C_4$-Monohydroxyalkylrest, einen $C_2$-$C_4$-Polyhydroxyalkylrest und einen ($C_1$-$C_4$)-Alkoxy-($C_1$-$C_4$)-alkylrest, einen $C_1$-$C_4$-Alkylrest, der durch eine Stickstoffgruppierung substituiert ist, Phenyl oder 4'-Aminophenyl darstellt;
$R_2$ ein Wasserstoffatom, einen $C_1$-$C_4$-Alkylrest, einen $C_1$-$C_4$-Monohydroxyalkylrest oder einen $C_2$-$C_4$-Polyhydroxyalkylrest, einen ($C_1$-$C_4$)-Alkoxy-($C_1$-$C_4$)-alkylrest oder einen $C_1$-$C_4$-Alkylrest, der durch eine Stickstoffgruppierung substituiert ist, darstellt;
$R_3$ ein Wasserstoffatom, ein Halogenatom, wie ein Chtoratom, einen $C_1$-$C_4$-Alkylrest, einen Sulforest, Carboxyrest, einen $C_1$-$C_4$-Monohydroxyalkylrest oder einen $C_1$-$C_4$-Hydroxyatkoxyrest, einen $C_1$-$C_4$-Acetylaminoalkoxyrest, einen $C_1$-$C_4$-Mesylaminoalkoxyrest oder einen $C_1$-$C_4$-Carbamoylaminoalkoxyrest darstellt;
$R_4$ ein Wasserstoffatom, ein Halogenatom oder einen $C_1$-$C_4$-Alkylrest darstellt;
$R_1$ und $R_2$ mit dem Stickstoffatom, das sie tragen, auch einen Stickstoffheterocyclus mit 5 oder 6 Kettengliedern bilden können, der gegebenenfalls mit einer oder mehreren Alkyl-, Hydroxy- oder Ureidogruppen substituiert ist.

**20.** Verfahren nach Anspruch 19, **dadurch gekennzeichnet, dass** die paraPhenylendiamine ausgewählt sind unter para-Phenylendiamin, para-Toluylendiamin, 2-Isopropyl-para-phenylendiamin, 2-β-Hydroxyethyl-para-phenylendiamin, 2,6-Dimethyl-para-phenylendiamin, N,N-Bis(β-hydroxyethyl)-para-phenylendiamin, 2-Chlor-para-phenylendiamin und ihren Additionssalzen mit einer Säure.

**21.** Verfahren nach Anspruch 18, **dadurch gekennzeichnet, dass** die Oxidationsbasen doppelte Basen sind, die der Formel (5) entsprechen:

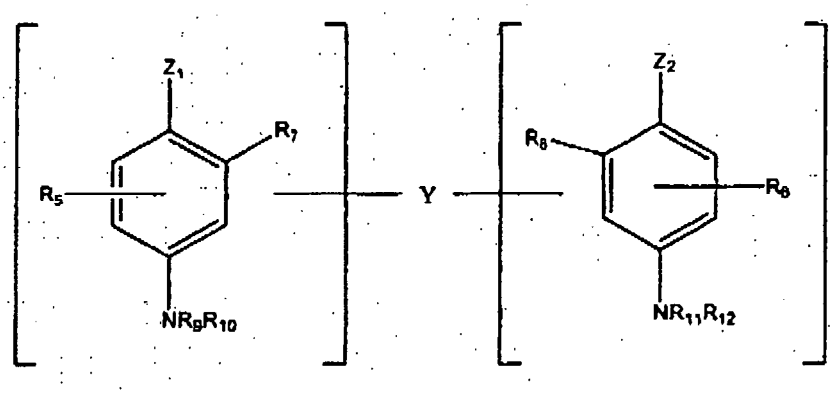

(5)

worin

$Z_1$ und $Z_2$, die identisch oder unterschiedlich sind, einen Hydroxylrest oder -$NH_2$-Rest, der mit einem $C_1$-$C_4$-Alkylrest oder mit einem Bindungsarm Y substituiert sein kann, darstellen;
der Bindungsarm Y eine Alkylenkette darstellt, die 1 bis 14 Kohlenstoffatome trägt, linear oder verzweigt ist, durch eine oder mehrere Stickstoffgruppierungen und/oder ein oder mehrere Heteroatome, wie die Atome Sauerstoff, Schwefel oder Stickstoff, unterbrochen oder terminiert sein kann und gegebenenfalls mit einem

oder mehreren Hydroxyl- oder $C_1$-$C_6$-Alkoxyresten substituiert sein kann;
$R_5$ und $R_6$ ein Wasserstoffatom oder Halogenatom, einen $C_1$-$C_4$-Alkylrest, einen $C_1$-$C_4$-Monohydroxyalkylrest, einen $C_2$-$C_4$-Polyhydroxyalkylrest, einen $C_1$-$C_4$-Aminoalkylrest oder einen Bindungsarm Y darstellen;
$R_7$, $R_8$, $R_9$, $R_{10}$, $R_{11}$ und $R_{12}$, die identisch oder unterschiedlich sind, ein Wasserstoffatom, einen Bindungsarm Y oder einen $C_1$-$C_4$-Alkylrest darstellen; wobei selbstverständlich ist, dass die Verbindungen der Formel (5) nur einen Bindungsarm Y pro Molekül tragen.

**22.** Verfahren nach Anspruch 21, **dadurch gekennzeichnet, dass** die doppelten Basen ausgewählt sind unter N,N'-Bis(β-hydroxyethyl)-N,N'-bis(4'-aminophenyl)-1,3-diaminopropanol, N,N'-Bis(β-hydroxyethyl)-N,N'-bis(4'-aminophenyl)ethylendiamin, N,N'-Bis(4-aminophenyl)tetramethylendiamin, N,N'-Bis(β-hydroxyethyl)-N,N'-bis(4-aminophenyl)tetramethylendiamin, N,N'-Bis(4-methylaminophenyl)tetramethylendiamin, N,N'-Bis(ethyl)-N,N'-bis(4'-amino-3'-methylphenyl)ethylendiamin, 1,8-Bis(2,5-diaminophenoxy)-3,5-dioxaoctan und ihren Additionssalzen mit einer Säure.

**23.** Verfahren nach Anspruch 18, **dadurch gekennzeichnet, dass** die Oxidationsbasen para-Aminophenole sind, die der Formel (6) entsprechen:

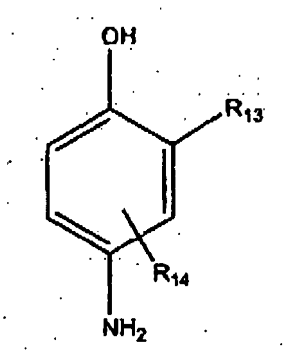

(6)

worin

- $R_{13}$ ein Wasserstoffatom, ein Halogenatom, wie z.B. Fluor, einen $C_1$-$C_4$-Alkylrest, einen $C_1$-$C_4$-Monohydroxyalkylrest, einen ($C_1$-$C_4$)-Alkoxy-($C_1$-$C_4$)-alkylrest oder einen $C_1$-$C_4$-Atninoalkylrest oder einen ($C_1$-$C_4$)-Hydroxyalkyl-($C_1$-$C_4$)-aminoalkylrest darstellt;
- $R_{14}$ ein Wasserstoffatom oder Halogenatom, wie z.B. Fluor, einen $C_1$-$C_4$-Alkylrest, einen $C_1$-$C_4$-Monohydroxyalkylrest, einen $C_2$-$C_4$-Polyhydroxyalkylrest, einen $C_1$-$C_4$-Aminoalkylrest, einen $C_1$-$C_4$-Cyanoalkylrest oder einen ($C_1$-$C_4$)-Alkoxy(-$C_1$-$C_4$)-alkylrest darstelt.

**24.** Verfahren nach Anspruch 23, **dadurch gekennzeichnet, dass** die para-Aminophenole ausgewählt sind aus para-Aminophenol, 4-Amino-3-methylphenal, 4-Amino-3-fluorphenol, 4-Amino-3-hydroxymethylphenol, 4-Amino-2-methylphenol, 4-Amino-2-hydroxymethylphenol, 4-Amino-2-methoxymethylphenol, 4-Amino-2-aminomethylphenol, 4-Amino-2-(β-hydroxyethylaminomethyl)phenol und ihren Additionssalzen mit einer Säure.

**25.** Verfahren nach Anspruch 18, **dadurch gekennzeichnet, dass** die Oxidationsbasen ortho-Diphenole sind, ausgewählt unter 2-Aminophenol, 2-Amino-1-hydroxy-5-methylbenzol, 2-Amino-1-hydroxy-6-methylbenzol, 5-Acetamido-2-aminophenol und ihren Additionssalzen mit einer Säure.

**26.** Verfahren nach Anspruch 18, **dadurch gekennzeichnet, dass** die Oxidationsbasen heterocyclische Basen sind, ausgewählt unter den Pyridinderivaten, den Pyrimidinderivaten, den Pyrazolderivaten und ihren Additionssalzen mit einer Säure.

**27.** Verfahren nach Anspruch 26, **dadurch gekennzeichnet, dass** die Pyridinderivate ausgewählt werden unter 2,5-Diaminopyridin, 2-(4-Methoicyphenyl)amino-3-aminopyridin, 2,3-Diamino-6-methoxypyridin, 2-(β-Methoxyethyl) amino-3-amino-6-methoxypyridin und 3,4-Diaminopyridin.

**28.** Verfahren nach Anspruch 26, **dadurch gekennzeichnet, dass** die Pyrimidinderivate ausgewählt werden unter

2,4,5,6-Tetraaminopyrimidin, 4-Hydroxy-2,5,6-triaminopyrimidin, 2-Hydroxy-4,5,6-triaminopyrimidin, 2,4-Dihydroxy-5,6-diaminopyrimidin, 2,5,6-Triaminopyrimidin und den Pyrazolopyrimidin-Derivaten, z.B. die, die in der Patentanmeldung FR-A-2 750 048 genannt sind und unter denen man Pyrazolo[1,5-a]pyrimidin-3,7-diamin; 2,5-Dimethylpyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo [1,5-a]pyrimidin-7-ylamino)ethanol, 2-(7-Aminopyrazolo[1,5-a]pyrimidin-3-ylamino)ethanol, 2-[(3-Aminopyrazolo [1,5-a]pyrimidin-7-yl)-(2-hydroxyethyl)amino]ethanol, 2-[(7-Aminopyrazolo[1,5-a]pyrimidin-3-yl)-(2-hydroxyethyl) amino]ethanol, 5,6-Dimethylpyrazolo[1,5-a]pyrimidin-3,7-diamin, 2,6-Dimethylpyrazolo[1,5-a]pyrimidin-3,7-diamin, 2,5-N7,N7-Tetramethylpyrazolo[1,5-a]pyrimidin-3,7-diamin, 3-Amino-5-methyl-7-imidazolylpropylaminopyrazolo [1,5-a]pyrimidin nennen kann, und ihren Additionssalzen und ihren tautomeren Formen, wenn ein Tautomeren-Gleichgewicht vorliegt.

**29.** Verfahren nach Anspruch 26, **dadurch gekennzeichnet, dass** die Pyrazolderivate ausgewählt werden unter 4,5-Diamino-1-methylpyrazol, 3,5-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-9-methylpyrazol, 4,5-Diamino-1-tert.-butyl-3-methylpyrazol, 4,5-Diamino-1-(β-hydroxyethyl)-3-methylpyrazol, 4,5-Diamino-1-(β-hydroxyethyl)pyrazol, 4,5-Diamino-l-ethyl-3-methylpyrazol, 4,5-Diamino-1-ethyl-3-(4'-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-S-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

**30.** Verfahren nach einem der Ansprüche 18 bis 29, **dadurch gekennzeichnet, dass** die Oxidationsbase 0,0005 bis 12 Gew.%, vorzugsweise 0,005 bis 8 Gew.-% des Gesamtgewichts der Zusammensetzung ausmacht.

**31.** Verfahren nach Anspruch 17, **dadurch gekennzeichnet, dass** die ortho-Diphenole einen Benzolring oder einen aromatischen kondensierten Ring umfassen, der wenigstens zwei Hydroxylgruppen an zwei aufeinander folgende Kohlenstoffatomen des Rings trägt.

**32.** Verfahren nach Anspruch 31, **dadurch gekennzeichnet, dass** die ortho-Diphenole Verbindungen der folgenden Formel sind:

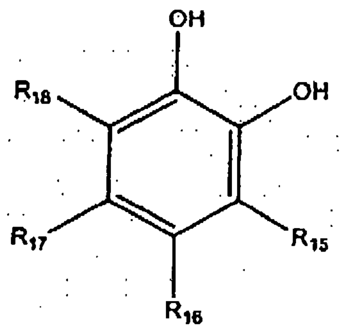

(7)

worin die Substituenten $R_{15}$ bis $R_{18}$, die identisch oder unterschiedlich sind, ein Wasserstoffatom, ein Halogenatom, einen Hydroxyl-, Carboxyl-, Alkylcarboxylat-, gegebenenfalls substituierten Amino-, gegebenenfalls substituierten linearen oder verzweigten Alkylrest, linearen oder verzweigten, gegebenenfalls substituierten Alkenylrest, gegebenenfalls substituierten Cycloalkyl-, Alkoxy-, Alkoxyalkyl-, Alkoxyarylrest, wobei die Arylgruppe gegebenenfalls substituiert sein kann, Aryl-, substituierten Arylrest, einen gegebenenfalls substituierten heterocyclischen Rest, einen Rest, der gegebenenfalls ein oder mehrere Siliciumatome enthält, darstellen oder zwei der Substituenten $R_{15}$ bis $R_{18}$ zusammen einen gesättigten oder ungesättigten Ring bilden können, der gegebenenfalls ein Heteroatom oder mehrere Heteroatome enthält und der gegebenenfalls mit einem oder mehreren gesättigten oder ungesättigten Ringen, die gegebenenfalls ein oder mehrere Heteroatome enthalten, kondensiert sein kann.

**33.** Verfahren nach Anspruch 31, **dadurch gekennzeichnet, dass** die ortho-Diphenole ausgewählt werden unter den

Flavanolen, Flavonolen, Anthocyaninidinen, Anthocyaninen, Hydroxybenzoaten, Flavonen, Iridoïden, wobei diese Verbindungen gegebenenfalls osyliert sein können und/oder in Form von Oligomeren sein können, den gegebenenfalls osylierten Hydroxystilbenen, 3,4-Dihydroxyphenylalanin und seinen Derivaten, 2,3-Dihydroxyphenylalanin und seinen Derivaten, 4,5-Dihydroxyphenylalanin und seinen Derivaten, 4,5-Dihydroxyindol und seinen Derivaten, 5,6-Dihydroxyindol und seinen Derivaten, 6,7-Dihydröxyindol und seinen Derivaten, 2,3-Dihydroxyindol und seinen Derivaten, den Dihydroxycinnamaten, den Hydroxycoumarinen, den Hydroxyisocoumarinen, den Hydroxycoumaronen, den Hydroxyisocoumaronen, den Hydroxychalconen, den Hydroxychromonen, den Anthocyanen, den Chinonen, den Hydroxyxanthonen und den Gemischen aus zwei oder mehr der vorangehenden Verbindungen.

**34.** Verfahren nach Anspruch 33, **dadurch gekennzeichnet, dass** die ortho-Diphenole ausgewählt werden unter 5,6-Dihydroxyindol und 5,6-Dihydroxyindolcarbonsäure.

**35.** Verfahren nach einem der Ansprüche 17 bis 34, **dadurch gekennzeichnet, dass** die ortho-Diphenole in Extrakten von Pflanzen, Früchten oder Zitrusfrüchten oder auch in Gemischen dieser Extrakte enthalten sind.

**36.** Verfahren nach Anspruch 35, **dadurch gekennzeichnet, dass** die ortho-Diphenole in Extrakten von Tee, Trauben, Apfel, Banane, Kartoffel oder auch in Gemischen dieser Extrakte enthalten sind.

**37.** Verfahren nach Anspruch 17, **dadurch gekennzeichnet, dass** die Kupplungsmittel ausgewählt werden unter den meta-Aminophenolen, den meta-Phenylendiaminen, den meta-Diphenolen, den Naphtholen und den heterocyclischen Kupplungsmitteln, z.B. den Indolderivaten, den Indolinderivaten, Sesamol und seinen Derivaten, den Pyridinderivaten, den Pyrazolotriazolderivaten, den Pyrazolonen, den Indazolen, den Benzimidazolen, den Benzothiazolen, den Benzoxazolen, den 1,3-Benzodioxolen, den Chinolinen und ihren Additionssalzen mit einer Säure.

**38.** Verfahren nach Anspruch 37, **dadurch gekennzeichnet, dass** die Kupplungsmittel ausgewählt werden unter 2,4-Diamino-1-(β-hydroxyethyloxy)benzol, 2-Methyl-5-aminophenol, 5-N-(β-Hydroxyethyl)amino-2-methylphenol, 3-Aminophenol, 1,3-Dihydroxybenzol, 1,3-Dihydroxy-2-methylbenzol, 4-Chlor-1,3-dihydroxybenzol, 2-Amino-4-(β-hydroxyethylamino)-1-methoxybenzol, 1,3-Diaminobenzol, 1,3-Bis(2,4-diaminophenoxy)propan, Sesamol, 1-Amino-2-methoxy-4,5-methylendioxybenzol, α-Naphthol, 6-Hydroxyindol, 4-Hydroxyindol, 4-Hydroxy-N-methylindol, 6-Hydroxyindolin, 2,6-Dihydroxy-4-methylpyridin, 1H-3-Methylpyrazol-5-on, 1-Phenyl-3-methylpyrazol-5-on, 2-Amino-3-hydroxypyridin, 3,6-Dimethylpyrazolo[3,2-c]-1,2,4-triazol, 2,6-Dimethylpyrazolo[1,5-b]-1,2,4-triazol und ihren Additionssalzen mit einer Säure.

**39.** Verfahren nach Anspruch 37 oder 38, **dadurch gekennzeichnet, dass** das Kupplungsmittel oder die Kupplungsmittel 0,0001 bis 15 Gew.-% des Gesamtgewichts der Zusammensetzung und vorzugsweise 0,001 bis 10% darstellt/darstellen.

**40.** Verfahren nach einem der Ansprüche 15 bis 39, **dadurch gekennzeichnet, dass** die Haarfärbezusammensetzung eine Direktfarbe oder mehrere Direktfarben umfasst, vorzugsweise ausgewählt unter den nitrierten, Azo- oder Anthrachinon-, neutralen, kationischen oder anionischen Farben.

**41.** Verfahren nach Anspruch 40, **dadurch gekennzeichnet, dass** die Direktfarbe oder die Direktfarben 0,001 bis 20 Gew.-%, vorzugsweise 0,01 bis 10 Gew.-%, des Gesamtgewichts der Zusammensetzung ausmacht/ausmachen.

**42.** Verfahren nach einem der Ansprüche 15 bis 41, **dadurch gekennzeichnet, dass** die Haarfärbezusammensetzung eine Aminosäure oder mehrere Aminosäuren und/oder ein Protein oder mehrere Proteine umfasst.

**43.** Verfahren nach Anspruch 42, **dadurch gekennzeichnet, dass** die Aminosäure oder die Aminosäuren wenigstens eine Thiolgruppe umfasst/umfassen und ausgewählt ist/sind unter den Aminosäuren, die eine Aminfunktion in α-Position zu einer Carbonsäurefunktion haben.

**44.** Verfahren nach Anspruch 43, **dadurch gekennzeichnet, dass** die Aminosäure(n) ausgewählt ist/sind unter Cystein und seinen Derivaten, die Proteine ausgewählt sind unter Glutathion und seinen Derivaten.

**45.** Verfahren nach einem der Ansprüche 40 bis 44, **dadurch gekennzeichnet, dass** das Molverhältnis der Aminosäure (oder der Aminosäuren) und des Proteins (oder der Proteine) zu der Oxidationsfarbe (den Oxidationsfarben) von 0,001 bis 50, vorzugsweise von 0,01 bis 5 und besser von 0,05 bis 2.5, variiert.

**46.** Verfahren nach einem der Ansprüche 15 bis 45, **dadurch gekennzeichnet, dass** die Haarfärbezusammensetzung außerdem ein Enzym umfasst.

**47.** Verfahren nach Anspruch 46, **dadurch gekennzeichnet, dass** das Enzym unter den Pyranoseoxidasen, den Glucoseoxidasen, den Glycerinoxidasen, den Lactatoxidasen, den Pyruvatoxidasen, den Uricasen, den Cholinoxidasen, den Sarcosinoxidasen, den Bilirubinoxidasen, den Laccasen, den Tyrosinasen, den Peroxidasen, den Katalasen, den Superoxiddismutasen und ihren Gemischen oder unter pflanzlichen oder tierischen Extrakten, die vorgenannte Enzyme enthalten, ausgewählt wird.

**48.** Verfahren nach Anspruch 47, **dadurch gekennzeichnet, dass** das Enzym unter den Tyrosinasen ausgewählt wird.

**49.** Verfahren nach Anspruch 47 oder 48, **dadurch gekennzeichnet, dass** die Haarfärbezusammensetzung $5 \cdot 10^{-3}$ bis 5 mg, vorzugsweise $5 \cdot 10^{-2}$ bis 0,5 mg, Enzym pro Milliliter Endzusammensetzung umfasst.

**50.** Verfahren nach einem der Ansprüche 15 bis 49, **dadurch gekennzeichnet, dass** die Oxidationsfarbe in einer Menge von 1 mM bis 10 mM pro Liter Zusammensetzung vorliegt.

**51.** Verfahren nach einem der Ansprüche 15 bis 50, **dadurch gekennzeichnet, dass** die Haarfärbezusammensetzung auch eine wirksame Menge eines Systems umfasst, umfassend einen ersten Bestandteil, ausgewählt aus den Salzen und Oxiden von Mn(II) und/oder Zn(II) und deren Gemischen, und einem zweiten Bestandteil, ausgewählt aus den Alkalihydrogencarbonaten, Erdalkalihydrogencarbonaten und ihren Gemischen, wobei die Verhältnisse des ersten und des zweiten Bestandteils wie folgt sind:

$$\frac{[Mn(II)]}{[HCO_3]} \leq 1, \text{ wobei } [Mn(II)] \neq 0$$

$$\frac{[Zn(II)]}{[HCO_3]} \leq 1, \text{ wobei } [Zn(II)] \neq 0$$

$$\frac{[Mn(II)+Zn(II)]}{[HCO_3]} \leq 1, \text{ wobei } [Mn(II)] \text{ und } [Zn(II)] \neq 0$$

worin [Mn(II)], [Zn(II)] und [$HCO_3$] die molaren Konzentrationen an Mn(II), Zn(II) und $HCO_3$ in der Zusammensetzung darstellen.

**52.** Verfahren nach Anspruch 51, **dadurch gekennzeichnet, dass** das Verhältnis $\frac{[Mn(II)]}{[HCO_3]}$ von $10^{-5}$ bis $10^{-1}$, vorzugswei-se von $10^{-3}$ bis $10^{-2}$, variiert und besser in der Größenordnung von $5 \cdot 10^{-3}$ liegt.

**53.** Verfahren nach Anspruch 51 oder 52, **dadurch** gekenn-zeichnet, dass das Verhältnis $\frac{[Zn(II)]}{[HCO_3]}$ von $10^{-4}$ bis 1, vorzugsweise von $10^{-3}$ bis 1, variiert und besser in der Größenordnung von 5. $10^{-1}$ liegt.

**54.** Verfahren nach einem der Ansprüche 51 bis 53, **dadurch gekennzeichnet, dass** das Verhältnis

$$\frac{[Mn(II) + Zn(II)]}{[HCO_3]}$$ von $10^{-5}$ bis $10^{-1}$, vorzugsweise von $10^{-3}$ bis $10^{-2}$, variiert.

**55.** Verfahren nach einem der Ansprüche 51 bis 53, **dadurch gekennzeichnet, dass** die Salze von Mn(II) und Zn(II) ausgewählt werden unter Chlorid, Fluorid, Iodid, Sulfat, Phosphat, Nitrat, Perchlorat, den Carbonsäuresalzen und ihren Gemischen.

**56.** Verfahren nach einem der Ansprüche 51 bis 55, **dadurch gekennzeichnet, dass** das Salz von Mn(II) und/oder Zn (II) das Chlorid ist.

**57.** Verfahren nach einem der Ansprüche 51 bis 56, **dadurch gekennzeichnet, dass** die Salze von Carbonsäuren Salze von hydroxylierten Carbonsäuren sind.

**58.** Verfahren nach einem der Ansprüche 51 bis 56, **dadurch gekennzeichnet, dass** das Salz einer hydroxylierten Carbonsäure das Gluconat ist.

**59.** Verfahren nach einem der Ansprüche 51 bis 58, **dadurch gekennzeichnet, dass** das Hydrogencarbonat unter Natriumhydrogencarbonat, Kaliumhydrogencarbonat und ihren Gemischen ausgewählt wird.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 5650171 A
- US 5700483 A
- US 5989527 A
- US 5833961 A
- US 4938951 A
- US 6001377 A
- US 2449070 A
- US 4592908 A
- US 5500218 A
- WO 9316678 A
- US 4545978 A
- GB 1026978 A
- GB 1153196 A
- DE 2359399
- JP 63169571 A
- JP 9110659 B
- WO 9615765 A
- FR 2750048 A
- DE 3843892
- DE 4133957
- WO 9408969 A
- WO 9408970 A
- FR 2733749 A
- DE 19543988
- FR 2694018 A
- FR 2112549 A
- EP 504005 A
- WO 9507988 A
- WO 9533836 A
- WO 9533837 A
- WO 9600290 A
- WO 9719998 A
- WO 9719999 A
- US 4367390 A
- EP 0863145 A
- EP 0517104 A
- EP 0570838 A
- EP 0796851 A
- EP 0775698 A
- EP 0878469 A
- EP 0933376 A
- EP 0893119 A
- EP 0669323 A
- US 2463264 A
- US 5237071 A
- US 5166355 A
- GB 2303549 A
- DE 19726184
- DE 19855649
- WO 9304665 A
- EP 0518772 A
- EP 0518773 A

### Littérature non-brevet citée dans la description

- *J. Pharm. Sci.,* 1978, vol. 67, 517-520
- *J. Pharm. Sci.,* 1979, vol. 68, 596-598
- *J. Controlled Release,* 1993, vol. 23, 247-260